# EUROPEAN PATENT APPLICATION

(11) **EP 4 548 892 A1**
(43) Date of publication of application: **07.05.2025**
(21) Application number: 23877060.6
(22) Date of filing: 12.09.2023
(51) Int. Cl.: A61F 13/72, A61F 13/56, A61F 13/62, A61F 13/76

(54) **WEARABLE ARTICLE HOUSING BODY, WEARABLE ARTICLE, AND ABSORBENT PAD**

(30) Priority: 12.10.2022 JP 2022164183
(71) Applicant: Unicharm Corporation, Ehime 799-0111 (JP)
(72) Inventor: SHIMBO, Yosuke, Kanonji-shi, Kagawa 769-1602 (JP); NASHIKI, Kento, Kanonji-shi, Kagawa 769-1602 (JP); TAKINO, Shunsuke, Kanonji-shi, Kagawa 769-1602 (JP); SAKURAI, Sayaka, Kanonji-shi, Kagawa 769-1602 (JP)
(74) Representative: Dolleymores
(86) International application number: PCT/JP2023/033233
(87) International publication number: WO 2024/080054

(57) **Abstract**

A wearable article housing body (90) comprises: a wearable article (1) to be used in combination with an absorbent pad (100); and a housing member (70) for housing said wearable article (1). The wearable article (1) has a hook member (FK) that engages the absorbent pad (100). The hook member (FK) is either a male hook member (FK1) or a female hook member (FK2). When a state in which the wearable article (1) is vertically stretched to twice the length from a natural state is defined as a double stretched state, the engaging force per unit area between the male hook member (FK1) and the female hook member (FK2) in a direction along the respective engaging faces is greater than a maximum tensile load per unit area required to stretch the wearable article (1) from the natural state to the double stretched state in a region that is located above the lower end of the hook member (FK) and that is located within 20 mm from the upper end of the wearable article (1).

## Description

### FIELD

The present invention relates to a wearable article housing body, a wearable article, and an absorbent pad.

### BACKGROUND

There are conventionally known underpants-shaped wearable articles that have stretchability and that are used in combination with an absorbent pad for absorbing excrement, such as urine, by attaching the pad to the wearable article's crotch portion. For example, Patent Document 1 discloses underpants (a wearable article) having stretchability and capable of being worn with an absorbent pad used in combination by engaging a hook-and-loop fastener (male member) provided on the absorbent pad to a hook-and-loop fastener (female member) provided on the inner surface of the underpants.

### [CITATION LIST]

### [PATENT LITERATURE]

[Patent Document 1] Japanese Patent Application Publication No. 2003-24385

### SUMMARY

### [TECHNICAL PROBLEM]

In cases where a wearable article constituted by a highly stretchable fabric is to be worn in a state where an absorbent pad is engaged with the wearable article, the wearable article is pulled upward by gripping the upper end portion thereof. At this time, if the force for pulling up the wearable article is greater than the engagement force, in the shearing direction, of a hook member that achieves engagement between the wearable article and the absorbent pad, then the engagement between the wearable article and the absorbent pad may become disengaged, making it difficult to pull up the absorbent pad.

The present invention was achieved in light of problems such as those described above, and an objective of an aspect of the invention is to make the engagement between an absorbent pad and a wearable article less likely to disengage when the wearable article is pulled up in a state where the absorbent pad is engaged therewith.

### [SOLUTION TO PROBLEM]

A main aspect of the invention for achieving the above-described objective is a wearable article housing body including: a wearable article having an up-down direction, a left-right direction, and a front-back direction intersecting with one another, the wearable article having stretchability in the up-down direction and the left-right direction and to be used in combination with an absorbent pad; and a housing member configured to house the wearable article. The wearable article is reusable. The housing member has an indication that calls to mind that the wearable article is to be used in combination with the absorbent pad. The wearable article includes a hook member configured to engage with the absorbent pad, the hook member being either one of a male hook member and a female hook member. A state in which the wearable article is taken out from the housing member and stretched in length in the up-down direction to twice the length from a natural state is defined as a double-stretched state. An engagement force, per unit area, between the male hook member and the female hook member in a direction along mutual engagement faces of the male hook member and the female hook member is greater than a maximum tensile load, per unit area, for when a region located above a lower end of the hook member up to 20 mm from an upper end of the wearable article is stretched from the natural state to the double-stretched state.

Other features of the present invention will become clear through the description of the present specification with reference to the accompanying drawings.

### [ADVANTAGEOUS EFFECT OF THE INVENTION]

According to the present invention, it is possible to make the engagement between an absorbent pad and a wearable article less likely to disengage when the wearable article is pulled up in a state where the absorbent pad is engaged therewith.

### BRIEF DESCRIPTION OF DRAWINGS

FIG. 1 is a schematic perspective view of a wearable article 1.
FIG. 2A is a plan view when the wearable article 1 in its natural state is viewed from the front side in the front-back direction.
FIG. 2B is a plan view when the wearable article 1 in its natural state is viewed from the back side in the front-back direction.
FIGS. 3A to 3C are diagrams illustrating a method for manufacturing the wearable article 1.
FIG. 4 shows a plan view and a cross-sectional view of an absorbent pad 100.
FIGS. 5A and 5B are diagrams illustrating a method for putting on a wearable article 2 of a comparative example when the wearable article 2 is used in combination with an absorbent pad 100.
FIGS. 6A to 6C are diagrams illustrating a method for putting on the wearable article 1 when the wearable article 1 is used in combination with an absorbent pad 100.
FIGS. 7A and 7B are diagrams illustrating a method for measuring the maximum tensile load at the time of stretching the wearable article 1 in the up-down direction.
FIGS. 8A to 8C are diagrams illustrating a method for measuring the engagement force between a male hook member and a female hook member.
FIG. 9 is a table showing the results and evaluations for when engagement force measurement was performed using a plurality of types of hook members.
FIG. 10 is a diagram illustrating a configuration of a hook member FK (engagement region 30).
FIG. 11 is a schematic perspective view illustrating an example of a wearable article housing body 90.
FIG. 12 is a schematic plan view illustrating a state in which both lateral ends, in the left-right direction, of a front-side waist region BR and back-side waist region BR of the wearable article 1 have been cut and the wearable article has been unfolded in the up-down direction.

### DESCRIPTION OF EMBODIMENTS

At least the following matters are disclosed in the description of this specification and the attached drawings.

### Aspect 1:

A wearable article housing body including: a wearable article having an up-down direction, a left-right direction, and a front-back direction intersecting with one another, the wearable article having stretchability in the up-down direction and the left-right direction and to be used in combination with an absorbent pad; and a housing member configured to house the wearable article, the wearable article being reusable, the housing member having an indication that calls to mind that the wearable article is to be used in combination with the absorbent pad, the wearable article including a hook member configured to engage with the absorbent pad, the hook member being either one of a male hook member and a female hook member, a state in which the wearable article is taken out from the housing member and stretched in length in the up-down direction to twice the length from a natural state being defined as a double-stretched state, an engagement force, per unit area, between the male hook member and the female hook member in a direction along mutual engagement faces of the male hook member and the female hook member being greater than a maximum tensile load, per unit area, for when a region located above a lower end of the hook member up to 20 mm from an upper end of the wearable article is stretched from the natural state to the double-stretched state.

According to the wearable article housing body of Aspect 1, during an action of putting on the wearable article having been taken out from the housing body, if the force, per unit area, in the direction along the engagement faces of the male hook member and the female hook member is stronger than the magnitude of the force, per unit area, when a predetermined portion (portion that is gripped and pulled with the hands) within the waist region is pulled in the up-down direction, the engagement between the hook members is likely to be easily maintained against the force pulling the wearable article. Thus, when the wearable article is pulled up in a state where an absorbent pad is attached thereto, the engagement between the absorbent pad and the wearable article can be made less likely to disengage.

### Aspect 2:

A wearable article having an up-down direction, a left-right direction, and a front-back direction intersecting with one another, the wearable article having stretchability in the up-down direction and the left-right direction and to be used in combination with an absorbent pad,
the wearable article including:
a hook member configured to engage with the absorbent pad, the hook member being either one of a male hook member and a female hook member,
a state in which the wearable article is stretched in length in the up-down direction to twice the length from a natural state being defined as a double-stretched state,
an engagement force, per unit area, between the male hook member and the female hook member in a direction along mutual engagement faces of the male hook member and the female hook member being greater than a maximum tensile load, per unit area, for when a region located above a lower end of the hook member up to 20 mm from an upper end of the wearable article is stretched from the natural state to the double-stretched state.

According to the wearable article of Aspect 2, during the putting-on action, if the force, per unit area, in the direction along the engagement faces of the male hook member and the female hook member is stronger than the magnitude of the force, per unit area, when a predetermined portion (portion that is gripped and pulled with the hands) within the waist region is pulled in the up-down direction, the engagement between the hook members is likely to be easily maintained against the force pulling the wearable article. Thus, when the wearable article is pulled up in a state where an absorbent pad is attached thereto, the engagement between the absorbent pad and the wearable article can be made less likely to disengage.

### Aspect 3:

The wearable article according to Aspect 2, wherein the engagement force, per unit area, in the direction along the mutual engagement faces between the male hook member and the female hook member is 13 mN/mm² or greater.

According to the wearable article of Aspect 3, when the wearable article, with the absorbent pad engaged therewith, is pulled up during the putting-on action of the wearable article, the engagement between the absorbent pad and the wearable article can be made less likely to disengage, compared to cases where the engagement force between the male hook member and the female hook member is less than 13 mN/mm².

### Aspect 4:

The wearable article according to Aspect 2 or 3, wherein the engagement force, per unit area, in the direction along the mutual engagement faces between the male hook member and the female hook member is 35 mN/mm² or greater.

According to the wearable article of Aspect 4, when the wearable article, with the absorbent pad engaged therewith, is pulled up during the putting-on action of the wearable article, the engagement between the absorbent pad and the wearable article can be made even less likely to disengage, compared to cases where the engagement force between the male hook member and the female hook member is less than 35 mN/mm².

### Aspect 5:

The wearable article according to any one of Aspects 2 to 4, including a main body portion constituted by a fabric whose maximum value of an elongation rate when a 50 mm × 200 mm sample piece is pulled at 5 N in the up-down direction or the left-right direction is 200% or greater.

According to the wearable article of Aspect 5, using such a highly stretchable fabric can improve the fit when the article is worn, and can also improve size conformability because the article can easily conform to the wearer's body size. Note that, even when the wearable article is stretched in the up-down direction during the putting-on action by using such a highly stretchable fabric, the engagement between the absorbent pad and the wearable article can be made less likely to disengage by using hook members that satisfy the conditions of Aspects 2 to 4.

### Aspect 6:

The wearable article according to any one of Aspects 2 to 5, wherein the hook member is the female hook member.

According to the wearable article of Aspect 6, since the female hook member does not have engagement protrusions and is softer than the male hook member, the female hook member is less likely to damage the wearer's skin when the wearable article including the same is worn. Further, when the wearable article is washed, the female hook member is less likely to damage the fabric constituting the main body portion of the wearable article.

### Aspect 7:

The wearable article according to any one of Aspects 2 to 6, wherein the female hook member is sewn onto a skin-side face of the wearable article.

According to the wearable article of Aspect 7, the female hook member can be suppressed from peeling off from the wearable article. Further, the female hook member is less likely to peel off even when the wearable article is washed. Further, since the female hook member can be attached to the wearable article without using an adhesive etc., the female hook member is safe to the human body and can easily offer a soft texture, even when it comes into contact with the wearer's skin.

### Aspect 8:

The wearable article according to any one of Aspects 2 to 7, including: a pair of leg openings; and a waist region located between an upper end of each of the leg openings and the upper end of the wearable article in the up-down direction, wherein the hook member is disposed in the waist region.

According to the wearable article of Aspect 8, the distance from the upper end of the wearable article to the hook member is shorter compared to a case where the hook member is disposed below the waist region (i.e., in the crotch region), and thus, the force for pulling up the waist region is conveyed efficiently to the hook member. Thus, the absorbent pad can be pulled upward more easily.

### Aspect 9:

The wearable article according to any one of Aspects 2 to 8, wherein in the up-down direction, a central position of the hook member is located below a central position of the waist region.

According to the wearable article of Aspect 9, by disposing the hook member below the central position of the waist region, the possibility that the hook member will be gripped with the hands will become low during the putting-on action. Thus, the engagement between the male hook member and the female hook member can be made less likely to peel apart.

### Aspect 10:

The wearable article according to any one of Aspects 2 to 9, including: a waist opening; and a waist low-stretch region in which a magnitude of force required for stretching the waist low-stretch region by a unit length along a peripheral edge portion of the waist opening is greater than a magnitude of force required for stretching another region by the unit length, wherein in the up-down direction, the hook member is disposed below the waist low-stretch region.

According to the wearable article of Aspect 10, disposing the hook member in a position separated from the waist low-stretch region (waist fold-back portion) helps to suppress the absorbent pad, which is engaged via the hook member, from being firmly pressed against the wearer's stomach or waist due to the strong contractive force exerted by the low-stretch region. Thus, it is possible to inhibit causing an uncomfortable feel to the wearer when the wearable article is worn.

### Aspect 11:

The wearable article according to any one of Aspects 2 to 10, wherein in the up-down direction, the hook member is disposed below a lower end of the waist low-stretch region by a distance greater than or equal to a width of the waist low-stretch region in the up-down direction.

According to the wearable article of Aspect 11, the hook member is less likely to be affected by the extension/contraction of the waist low-stretch region, compared to cases where the distance between the hook member and the waist low-stretch region is shorter than the width of the waist low-stretch region. This helps to further suppress the absorbent pad, which is engaged via the hook member, from being pressed against the wearer's skin side, and thus it is possible to inhibit causing an uncomfortable feel to the wearer.

### Aspect 12:

The wearable article according to any one of Aspects 2 to 11, wherein in the up-down direction, a central position of the hook member is located above a central position between a central position of the waist region and a lower end of the waist region.

According to the wearable article of Aspect 12, the distance from the upper end of the wearable article is closer compared to the case where the hook member is located below the central position between the central position of the waist region and the lower end of the waist region in the up-down direction, and thus, the force for pulling the waist region upward is conveyed easily to the hook member. Further, the distance from the waist low-stretch region is farther compared to the case where the hook member is located above the central position of the waist region in the up-down direction, and thus, the influence caused by the strong contractive force in the waist low-stretch region is suppressed. Thus, it is easy to pull up the absorbent pad, and It is also possible to inhibit causing an uncomfortable feel to the wearer.

### Aspect 13:

The wearable article according to any one of Aspects 2 to 12, including a pair of leg openings, wherein in the natural state, an upper end of each of the leg openings is located above a lower end of the wearable article in the up-down direction at a central position in the left-right direction.

According to the wearable article of Aspect 13, when the wearable article has an underpants-like shape wherein the leg openings are cut obliquely upward, the leg openings are less likely to get caught on the wearer's legs compared to cases where the wearable article has a boxer-shorts shape, and the entire wearable article is easy to pull up smoothly. Thus, the waist region is suppressed from being stretched excessively in the up-down direction, thereby reducing the shearing force exerted on the engagement portion with the absorbent pad. In this way, the engagement between the absorbent pad and the wearable article can be made less likely to disengage.

### Aspect 14:

The wearable article according to any one of Aspects 2 to 13, wherein in the natural state, a peripheral edge of each of the leg openings in a front side in the front-back direction has a shape that is narrowed inwardly in the left-right direction.

According to the wearable article of Aspect 14, when the wearer moves his/her leg toward the front side while wearing the wearable article, the leg opening is less likely to interfere with the wearer's leg, and it is possible to suppress an unnatural feel from occurring while the wearable article is worn. Further, during the putting-on action, the leg openings are less likely to get caught on the wearer's legs when the wearer's legs are passed through the leg openings and the wearable article is pulled upward. Thus, the waist region is suppressed from being stretched in the up-down direction, thereby reducing the shearing force exerted on the engagement portion with the absorbent pad. In this way, the engagement between the absorbent pad and the wearable article can be made less likely to disengage.

### Aspect 15:

The wearable article according to any one of Aspects 2 to 14, wherein a length, in the left-right direction, of the crotch region in the natural state is from 25% to 50% of a length, in the left-right direction, of the wearable article in the natural state.

According to the wearable article of Aspect 15, during the putting-on action, the leg opening can easily be made wider than the wearer's leg, and thus the leg can be passed through easily. Therefore, the wearable article can easily be pulled up smoothly, and thus the waist region is made less likely to stretch in the up-down direction. Thus, force (shearing force) in the direction along the hook members' engagement faces is less likely to be exerted, and the engagement between the absorbent pad and the wearable article can be made less likely to disengage.

### Aspect 16:

The wearable article according to any one of Aspects 2 to 15, including stretch-suppressing means having a predetermined length in the up-down direction.

According to the wearable article of Aspect 16, in a portion where the stretch-suppressing means is provided, stretching in the up-down direction is suppressed, thus making it less likely for a force (shearing force) to be exerted in the direction along the engagement faces of the hook members that engage the wearable article and the absorbent pad. In this way, the engagement between the absorbent pad and the wearable article can be made less likely to disengage.

### Aspect 17:

The wearable article according to any one of Aspects 2 to 16, wherein in the up-down direction, there is a portion in which the stretch-suppressing means and the hook member overlap one another.

According to the wearable article of Aspect 17, in the portion in which the stretch-suppressing means and the hook member overlap one another, the fabric constituting the wearable article is less likely to stretch in the up-down direction. Thus, the force (shearing force) exerted with respect to the engagement faces of the hook members in the direction along the engagement faces is further reduced. In this way, the engagement between the absorbent pad and the wearable article can be made even less likely to disengage.

### Aspect 18:

The wearable article according to any one of Aspects 2 to 17, including: a waist opening; and a waist low-stretch region in which a magnitude of force required for stretching the waist low-stretch region by a unit length along a peripheral edge portion of the waist opening is greater than a magnitude of force required for stretching another region by the unit length, wherein in the up-down direction, the stretch-suppressing means is continuous from the waist low-stretch region to the hook member.

According to the wearable article of Aspect 18, the waist low-stretch region and the hook member are vertically coupled by the stretch-suppressing means, and thereby, the mutually coupled portions function like a skeletal frame in the waist region of the wearable article and help to suppress stretching in the up-down direction. That is, during the putting-on action, the region that is gripped and pulled up with the hands is less likely to stretch in the up-down direction. Thus, force in the direction along the engagement faces of the hook members is less likely to be exerted, and the engagement between the absorbent pad and the wearable article can be made even less likely to disengage.

### Aspect 19:

The wearable article according to any one of Aspects 2 to 18, wherein the stretch-suppressing means includes a central stretch-suppressing portion located in a central portion in the left-right direction, and a pair of lateral stretch-suppressing portions located respectively on both sides of the central stretch-suppressing portion in the left-right direction.

According to the wearable article of Aspect 19, when both lateral portions of the waist region are gripped and pulled upward during the putting-on action, the lateral stretch-suppressing portions make it less likely for both lateral portions to be stretched in the up-down direction. Further, since also the central stretch-suppressing portion is provided in the central portion, the waist region, in its entirety, becomes less likely to be stretched in the up-down direction. Thus, force (shearing force) in the direction along the hook members' engagement faces is less likely to be exerted, and the engagement between the absorbent pad and the wearable article can be made less likely to disengage.

### Aspect 20:

The wearable article according to any one of Aspects 2 to 19, including a pair of leg openings, wherein each of the pair of leg openings includes an overcast-stitched portion along a peripheral edge thereof, and there is a portion in which the lateral stretch-suppressing portion and the overcast-stitched portion intersect with one another.

According to the wearable article of Aspect 20, during the putting-on action, the force applied by gripping and pulling up both lateral portions of the waist region with the hands can be conveyed easily to the overcast-stitched portion along the peripheral edge of the leg openings via the lateral stretch-suppressing portions, and the leg openings can be pulled upward easily. Thus, the leg openings are less likely to get caught on the wearer's legs, and the wearable article can be pulled up smoothly, making it less likely for the waist region to be stretched in the up-down direction. In this way, force (shearing force) in the direction along the hook members' engagement faces is less likely to be exerted, and the engagement between the absorbent pad and the wearable article can be made less likely to disengage.

### Aspect 21:

The wearable article according to any one of Aspects 2 to 20, including: a pair of leg openings; a crotch region located between an upper end of each of the leg openings and a lower end of the wearable article in the up-down direction and between respective inner ends of the pair of leg openings in the left-right direction; and a waist region located between the upper end of each of the leg openings and the upper end of the wearable article in the up-down direction, wherein in the natural state, a first imaginary line has no portion overlapping the hook member, the first imaginary line connecting: an end on an upper side of the waist region on one side in the left-right direction; and an end on a lower side of the crotch region on the one side in the left-right direction.

According to the wearable article of Aspect 21, during the putting-on action, when both lateral portions of the waist region are gripped and pulled upward with the hands, tension occurring along the first imaginary line is suppressed from being exerted in the direction along the hook members' engagement face. Thus, the engagement between the absorbent pad and the wearable article can be made less likely to disengage.

### Aspect 22:

The wearable article according to any one of Aspects 2 to 21, including a central stretch-suppressing portion located in a central portion in the left-right direction and having a predetermined length in the up-down direction, wherein in the natural state, a second imaginary line has no portion overlapping the hook member, the second imaginary line connecting: the end on the upper side of the waist region on the one side in the left-right direction; and a lower end of the central stretch-suppressing portion.

According to the wearable article of Aspect 22, during the putting-on action, when both lateral portions of the waist region are gripped and pulled upward with the hands, tension occurring along the second imaginary line is suppressed from being exerted in the direction along the hook members' engagement face. Thus, the engagement between the absorbent pad and the wearable article can be made less likely to disengage.

### Aspect 23:

The wearable article according to any one of Aspects 2 to 22, including the central stretch-suppressing portion located in the central portion in the left-right direction and having the predetermined length in the up-down direction, wherein in the natural state, a third imaginary line has no portion overlapping the hook member, the third imaginary line connecting: the end on the upper side of the waist region on the one side in the left-right direction; and an end on the lower side of the crotch region on the other side in the left-right direction.

According to the wearable article of Aspect 23, during the putting-on action, when both lateral portions of the waist region are gripped and pulled upward with the hands, tension occurring along the third imaginary line is suppressed from being exerted in the direction along the hook members' engagement face. Thus, the engagement between the absorbent pad and the wearable article can be made less likely to disengage.

### Aspect 24:

An absorbent pad to be used in combination with the wearable article according to any one of Aspects 2 to 23, the absorbent pad having a longitudinal direction, a lateral direction, and a thickness direction intersecting with one another in a stretched state, the absorbent pad including: a liquid-absorbent absorbent body; and an other hook member of either one of the male hook member and the female hook member.

According to the absorbent pad of Aspect 24, by providing the other hook member which easily engages with the one hook member provided on the wearable article, the absorbent pad can be engaged stably with the wearable article. Thus, even when the wearable article is pulled upward with the absorbent pad engaged therewith, the engagement between the two can be made less likely to disengage.

### Aspect 25:

The absorbent pad according to Aspect 24, wherein when viewed in the thickness direction, the hook member has a portion overlapping the absorbent body.

According to the absorbent pad of Aspect 25, by overlapping the absorbent body which has high rigidity, deformation of the hook member in the thickness direction is suppressed, and the hook member is easily maintained in a planar state. Thus, it is possible to facilitate engagement with the wearable article's hook member. Also, by being able to easily maintain the hook member in a planar state, it is possible to suppress the engagement from coming off (peeling) due to curl-up of the end portion, in the up-down direction, of the hook member when the hook member, which is in engagement with the wearable article, is pulled upward. In this way, the engagement between the absorbent pad and the wearable article can be made less likely to disengage.

### Aspect 26:

The absorbent pad according to Aspect 24 or 25, wherein the hook member is joined to the absorbent pad by using an adhesive, and there is no dry edge along a peripheral edge of the hook member.

According to the absorbent pad of Aspect 26, the adhesive is applied to the entire joining face of the hook member and no dry edge is formed, and thus, peeling etc. is suppressed. Thus, the engagement between the absorbent pad and the wearable article can be made less likely to disengage.

### Aspect 27:

The absorbent pad according to any one of Aspects 24 to 26, wherein a length in the longitudinal direction is 300 mm or greater, and a length in the lateral direction is 80 mm or greater.

According to the absorbent pad of Aspect 27, when the length in the lateral direction is 80 mm or greater, the width of the hook member can be made wider compared to the opposite case, and thus, the hook member can engage more firmly with the wearable article. Further, when the length in the longitudinal direction is 300 mm or greater, it is easier to secure a sufficient area for retaining liquid-absorbent fiber etc. necessary for achieving water absorbency, compared to the opposite case.

### Aspect 28:

The wearable article according to any one of Aspects 2 to 27, wherein the fabric constituting the wearable article contains at least 90 wt% of polyester or polyethylene.

According to the wearable article of Aspect 28, since polyester and polyethylene are materials having a high degree of elongation compared to cotton, containing a large amount of such materials can further facilitate elongation. Further, polyester and polyethylene are materials having non-water absorbency and quick dryability, and thus, the dryability of the wearable article is improved.

### Aspect 29:

The wearable article according to any one of Aspects 2 to 28, wherein the fabric constituting the wearable article does not contain any water-absorbent material.

According to the wearable article of Aspect 29, by not containing any water-absorbent material, the fabric is less likely to absorb water. Thus, the time required for drying after washing can be reduced, leading to an improvement in the dryability of the wearable article.

### Aspect 30:

The wearable article according to Aspect 28 or 29, wherein the fabric constituting the wearable article contains less than 10 wt% of a material having higher stretchability than polyester or polyethylene.

According to the wearable article of Aspect 30, since the fabric constituting the wearable article contains 90 wt% or more of polyester or polyethylene, by including the aforementioned material having higher stretchability in the remainder accounting for less than 10 wt%, it is possible to improve stretchability while securing good dryability.

### Aspect 31:

The wearable article according to any one of Aspects 2 to 30, wherein the fabric constituting the wearable article includes a region that contains a material having higher stretchability than polyester or polyethylene and a region that does not contain the same.

According to the wearable article of Aspect 30, by disposing the highly stretchable material in a region where fit is particularly needed (i.e., by forming the region containing the material having higher stretchability), the fit can be further improved in a limited area of the wearable article, thereby improving functionality. Alternatively, in addition to improving the fit in a certain area, for example, by providing regions containing the material having higher stretchability in a plurality of areas with intervals therebetween, the fit of the wearable article can be improved in its entirety.

### Aspect 31:

The wearable article according to any one of Aspects 2 to 30, wherein the engagement region is provided with a separate sheet member that is different from a knitted fabric constituting the main body portion, and the separate sheet member is constituted by polyethylene or polyester.

According to the wearable article of Aspect 31, since the separate sheet member is joined to the main body portion, by making the separate sheet member from a material having quick dryability like the main body portion, the dryability of the wearable article is improved.

### Aspect 32:

The wearable article according to Aspect 31, wherein the separate sheet member is joined to the fabric constituting the wearable article by being sewn with a thread along a peripheral edge portion of the separate sheet member.

According to the wearable article of Aspect 32, in a region on the inner side of the peripheral edge portion, which has been sewn with a thread, the fabric and the separate sheet member are not in tight adhesion (not joined) in the thickness direction, and there is a space formed between the separate sheet member and the fabric. That is, the entire separate sheet member is not in a state joined to the fabric, and therefore, compared to a case in which the entire sheet member is joined thereto, the face of the separate sheet member in contact with the fabric has a wide area in contact with air, which makes it easy for moisture to evaporate. Such a structure improves dryability.

### Aspect 33:

The wearable article according to Aspect 31 or 32, wherein, when viewed in the thickness direction, there is a portion in which the separate sheet member and the stretch-suppressing portion overlap one another.

According to the wearable article of Aspect 33, by providing the stretch-suppressing portion, a change in contractive force occurs in the fabric constituting the wearable article, and a space is more likely to be formed between the skin-side face of the fabric and the non-skin-side face of the separate sheet member. Dryability is improved by allowing air to easily escape from such a space.

### Aspect 34:

The wearable article according to any one of Aspects 31 to 33, wherein, as regards the size of the separate sheet member, the length in the up-down direction is 50 mm or less, and the length in the left-right direction is 150 mm or less.

According to the wearable article of Aspect 34, if the separate sheet member is too large, the sheet member portion may become difficult to dry, but by adjusting the size within the aforementioned range, it becomes easier to dry while securing an area that can maintain sufficient engagement force with the fabric.

### Aspect 35:

The wearable article according to any one of Aspects 31 to 34, wherein the thickness of the separate sheet member is 3 mm or less.

According to the wearable article of Aspect 35, since the sheet member is not too thick, it is possible to maintain easy dryability while securing a thickness capable of maintaining sufficient engagement force with the fabric.

### Aspect 36:

The wearable article according to any one of Aspects 2 to 35, wherein, in a state in which both lateral ends, in the left-right direction, of the front-side waist region and back-side waist region of the wearable article have been cut and the wearable article has been unfolded in the up-down direction, the area of region S1 is smaller than the area of the remaining region S2, where the region S1 is a region between an upper end of the front-side engagement region and an upper end of the back-side engagement region in the up-down direction and between the respective ends of the front-side engagement region and back-side engagement region most toward one side and the respective ends of the front-side engagement region and back-side engagement region most toward the other side in the left-right direction.

According to the wearable article of Aspect 36, the region S1 within the fabric constituting the wearable article is the region that is covered by the absorbent pad when the absorbent pad is attached. Therefore, if drying is somewhat insufficient after washing the wearable article, or if for some other reason the wearable article gets wet after attaching the absorbent pad, the region S1 is likely to have low dryability thereafter because it is covered by the absorbent pad. However, by making the area of the region S1 smaller than the area of the region S2 in the fabric constituting the wearable article, it is easier to suppress deterioration of dryability of the entire wearable article, compared to the opposite case.

### Aspect 37:

The wearable article according to any one of Aspects 2 to 35, wherein, in a state in which both lateral ends, in the left-right direction, of the front-side waist region and back-side waist region of the wearable article have been cut and the wearable article has been unfolded in the up-down direction, the area of region S1 is larger than the area of the remaining region S2, where the region S1 is a region between an upper end of the front-side engagement region and an upper end of the back-side engagement region in the up-down direction and between the respective ends of the front-side engagement region and back-side engagement region most toward one side and the respective ends of the front-side engagement region and back-side engagement region most toward the other side in the left-right direction.

According to the wearable article of Aspect 37, even if drying is somewhat insufficient after washing the wearable article, the region S1 will be covered by the absorbent pad, and thus, the skin is less likely to become wet and wearing comfort is less likely to be impaired.

### EMBODIMENTS:

An underpants-shaped wearable article 1 of a type that can be used in combination with an absorbent pad 100 for absorbing excrement, such as urine, by attaching the pad to the interior of the wearable article (also referred to hereinafter simply as "wearable article 1") is described as an example of a wearable article according to an embodiment of the present invention.

### Basic Configuration of Wearable Article 1:

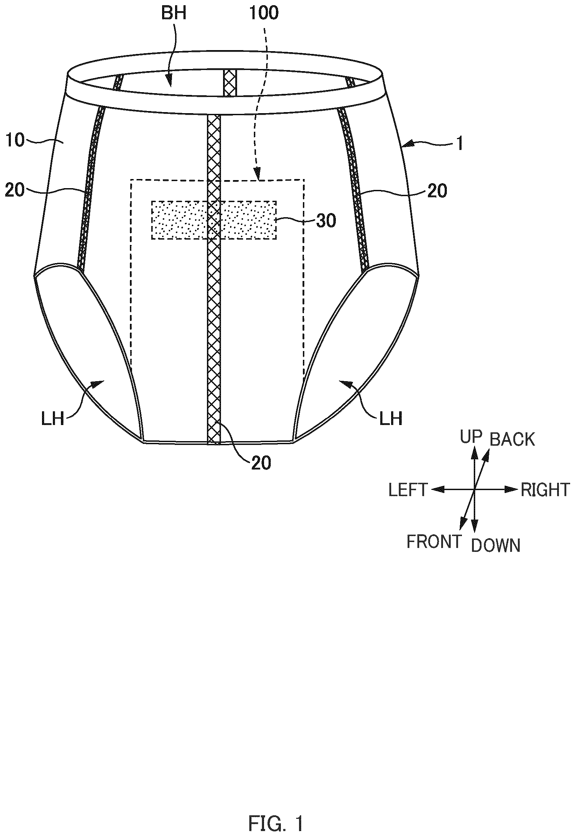

FIG. 1 is a schematic perspective view of a wearable article 1 according to the present embodiment. In an underpants-shaped state as illustrated in FIG. 1, the wearable article 1 has an "up-down direction", a "left-right direction", and a "front-back direction" orthogonal to one another. In the up-down direction, in a state where the wearable article 1 is worn by a wearer, "upper side (up)" refers to the side toward the wearer's waist, and "lower side (down)" refers to the side toward the wearer's crotch area. In the front-back direction, "front side" refers to the wearer's stomach side, and "back side" refers to the wearer's back side in a worn state. Each material (sheet member) constituting the wearable article 1 has a "thickness direction". In the thickness direction, in a state where the wearable article 1 is worn by a wearer, "skin side" refers to the side closer to the wearer's skin, and "non-skin side" refers to the opposite side therefrom.

The wearable article 1 of the present embodiment is formed in an underpants shape including a waist opening BH provided on the upper side in the up-down direction, and a pair of leg openings LH, LH provided on the lower side in the up-down direction and respectively on both sides in the left-right direction. An absorbent pad 100 can be detachably attached to the inside of the underpants-shaped wearable article, and by changing absorbent pads 100, the wearable article 1 itself can be used (worn) repeatedly. For example, when urine etc. is excreted in a state where a wearer (user) is wearing the wearable article 1 having an absorbent pad 100 attached thereto, the absorbent pad 100 soiled as a result of absorbing excrement can be removed from the wearable article 1 and replaced with a new absorbent pad 100, so that the wearable article 1 can be reused. Further, the wearable article 1 can be washed and can thus be used hygienically over a long period of time (such as 2 weeks or longer).

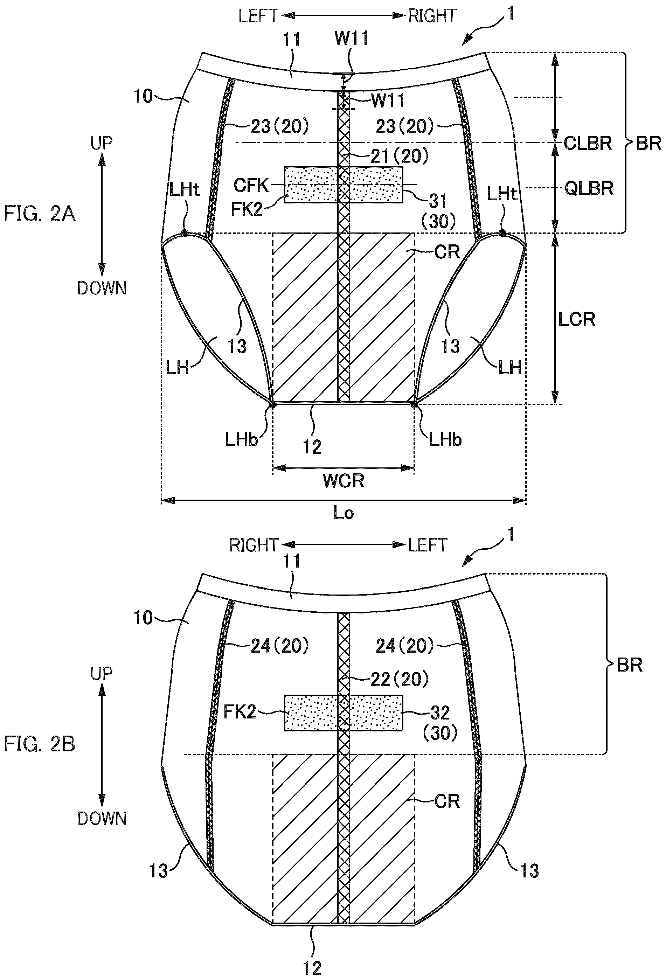

FIG. 2A is a plan view when the wearable article 1 in its natural state is viewed from the front side in the front-back direction. FIG. 2B is a plan view when the wearable article 1 in its natural state is viewed from the back side in the front-back direction.

Herein, "natural state" refers to a state in which the wearable article 1 has been left to stand for a predetermined time. For example, a folded-and-packaged wearable article 1 is taken out from its package and is then spread out gently so as not to cause deformation and is placed on a flat surface such as a desk. "Natural state" refers to a state in which the wearable article 1 has been left flat on the flat surface for 5 minutes such that there are almost no creases remaining on the surface thereof.

The wearable article 1 includes a main body portion 10, stretch-suppressing portions 20, and engagement regions 30.

The main body portion 10 is a part that constitutes the exterior of the wearable article 1, and is formed by a sheet member having stretchability. In the present embodiment, the sheet member forming the main body portion 10 is constituted by a "woven fabric" or a "knitted fabric" and has stretchability in both the up-down direction and the left-right direction. Note that a "woven fabric" is a fabric made into a planar configuration by orthogonally interlacing weft threads through a multitude of warp threads arranged in parallel, and an example thereof includes a fabric called *"fuhaku"* (a type of woven fabric). In the case of woven fabrics, it is possible to form woven fabrics having stretchability by using highly stretchable threads or by varying the thread density. On the other hand, a "knitted fabric" is a fabric made into a planar configuration by forming loops with a thread and intermeshing the loops with other loops, and an example thereof includes a fabric called "knit". Since a knitted fabric is made by intermeshing a multitude of loops, the fabric can be extended/contracted greatly with even a small force by deforming the loops. The following describes an example in which the main body portion 10 (wearable article 1) is made by using a knitted fabric.

The main body portion 10 is constituted by a so-called circular knitted fabric which is knitted into a tube shape along the up-down direction. A portion of the upper end portion of the tubular main body portion 10 is folded back from above toward below in the up-down direction, and thereby a waist fold-back portion 11 is formed. This waist fold-back portion 11 suppresses fabric fraying at the upper end portion (i.e., waist opening BH) of the circular knitted fabric. Further, threads that are less prone to extension/contraction than those in other parts are knitted in at the waist fold-back portion 11. This increases the contractive force along the peripheral edge of the waist opening BH, and thus, when the wearable article 1 is worn, the waist opening BH can be suppressed from sagging down and the fit can be improved. Note that, instead of folding back the upper end portion of the circular knitted fabric, a separate sheet member may be layered and joined onto the upper end portion to exert a large contractive force along the peripheral edge of the waist opening BH.

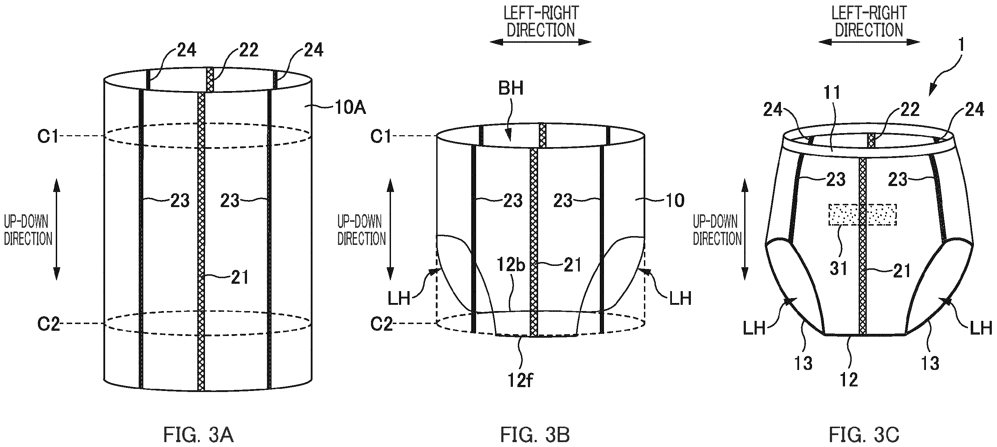

On the other hand, at the lower end portion of the main body portion 10, a crotch sewn portion 12 is formed by sewing together the front side and the back side of the circular knitted fabric. By joining the lower end portion of the main body portion 10 by the crotch sewn portion 12, the tube-shaped circular knitted fabric is formed into an underpants shape having a pair of leg openings LH, LH (see FIG. 3 described further below). In the present embodiment, the crotch sewn portion 12 is located at the lowermost position of the wearable article 1 in the up-down direction (see FIGS. 2A and 2B).

The pair of leg openings LH, LH is formed by cutting off portions of the main body portion 10 (see FIG. 3). A leg-opening overcast-stitched portion 13 is formed in the peripheral edge portion of each leg opening LH, LH. The leg-opening overcast-stitched portion 13 is formed by wrapping and stitching a thread around the edge of the knitted fabric along the peripheral edge of the leg opening LH. This helps to suppress fabric fraying at the cut-off section of the leg opening LH. Further, by forming the leg-opening overcast-stitched portions 13, the fit of the leg openings LH can be improved while the wearable article is worn, and side leakage of excrement, such as urine, can be suppressed easily.

As illustrated in FIGS. 2A and 2B, in the up-down direction of the wearable article 1 in a natural state, the upper end of the leg opening LH is defined as LHt. The upper end LHt is located slightly inward from the outermost end in the left-right direction. Similarly, in the up-down direction of the wearable article 1 in a natural state, the lower end of the leg opening LH is defined as LHb. The lower end LHb is located at the lowermost position of the wearable article 1 in the up-down direction and at the innermost position of the leg opening LH in the left-right direction.

In the wearable article 1, "crotch region CR" is defined as a region, shown in hatching, between the upper end LHt of the leg opening LH and the lower end LHb thereof (i.e., the lower end of the wearable article 1) in the up-down direction and between the respective lower ends LHb, LHb of the pair of leg openings LH (i.e., between the respective inner ends of the leg openings LH) in the left-right direction. The crotch region CR is a region located primarily in the crotch area of a wearer (user) when the wearable article 1 is worn, and is also a region that retains at least a portion of the absorbent pad 100 when the absorbent pad 100 is attached. Further, in the wearable article 1, "waist region BR" is defined as a region located between the upper end LHt of the leg opening LH and the upper end of the wearable article 1 in the up-down direction. The waist region BR is a region located primarily in the waist portion (abdomen, buttocks, and hip) of a wearer (user) when the wearable article 1 is worn.

The stretch-suppressing portion 20 is a portion having a function as stretch-suppressing means configured to suppress extension/contraction of the wearable article 1 in the up-down direction. In the present embodiment, a plurality of stretch-suppressing portions 20, each having a predetermined length in the up-down direction, are provided in predetermined positions of the main body portion 10 of the wearable article 1. More specifically, on the front side of the wearable article 1 (main body portion 10) in the front-back direction, a front-side central stretch-suppressing portion 21 is provided in a central portion in the left-right direction, and a pair of front-side lateral stretch-suppressing portions 23, 23 (corresponding to lateral-portion stretch-suppressing means) is provided respectively on both sides of the front-side central stretch-suppressing portion 21 in the left-right direction (see FIG. 2A). Similarly, on the back side of the wearable article 1 (main body portion 10) in the front-back direction, a back-side central stretch-suppressing portion 22 is provided in a central portion in the left-right direction, and a pair of back-side lateral stretch-suppressing portions 24, 24 (lateral-portion stretch-suppressing means) is provided respectively on both sides of the back-side central stretch-suppressing portion 22 in the left-right direction (see FIG. 2B).

When the wearable article 1 is to be put on by a wearer (user), actions are performed wherein the wearer's legs are passed through the pair of leg openings LH and then the waist region BR is gripped and the wearable article is pulled up to the crotch area. As described above, the main body portion 10 is formed by a knitted fabric (or a woven fabric) having stretchability. Therefore, if the main body portion 10 stretches excessively in the up-down direction during the action of pulling up the wearable article 1, it may become difficult to pull up the wearable article 1. In contrast, in the wearable article 1 of the present embodiment, the parts where the stretch-suppressing portions 20 are provided suppress extension/contraction (stretching) of the main body portion 10 in the up-down direction, and thus, the wearable article 1 can be pulled upward easily.

The stretch-suppressing portion 20 (stretch-suppressing means) is achieved by applying, to the knitted fabric constituting the main body portion 10, a process that reduces the stretchability of the knitted fabric along the up-down direction. The stretchability in the up-down direction can be reduced, for example, by applying a process of sewing (or "stitching") a thread having low stretchability into a predetermined region of the knitted fabric constituting the main body portion 10. Alternatively, the stretchability may be reduced in a portion of the knitted fabric by changing the knitting pattern in predetermined parts or by changing the stretchability of the thread during knitting of the knitted fabric.

Other than the above, the stretchability of the knitted fabric may be reduced by welding or pressure-bonding a member such as silicone or rubber, or the stretchability may be reduced by applying a compression process (embossing etc.) to predetermined parts within the knitted fabric.

The engagement region 30 is a region in which an absorbent pad 100 can be detachably attached to the wearable article 1, and is provided on the skin side of the main body portion 10 in the thickness direction. In the present embodiment, a rectangular front-side engagement region 31 is provided on the skin-side face of the waist region BR on the front side in the front-back direction (see FIG. 2A), and a rectangular back-side engagement region 32 is provided on the skin-side face of the waist region BR on the back side in the front-back direction.

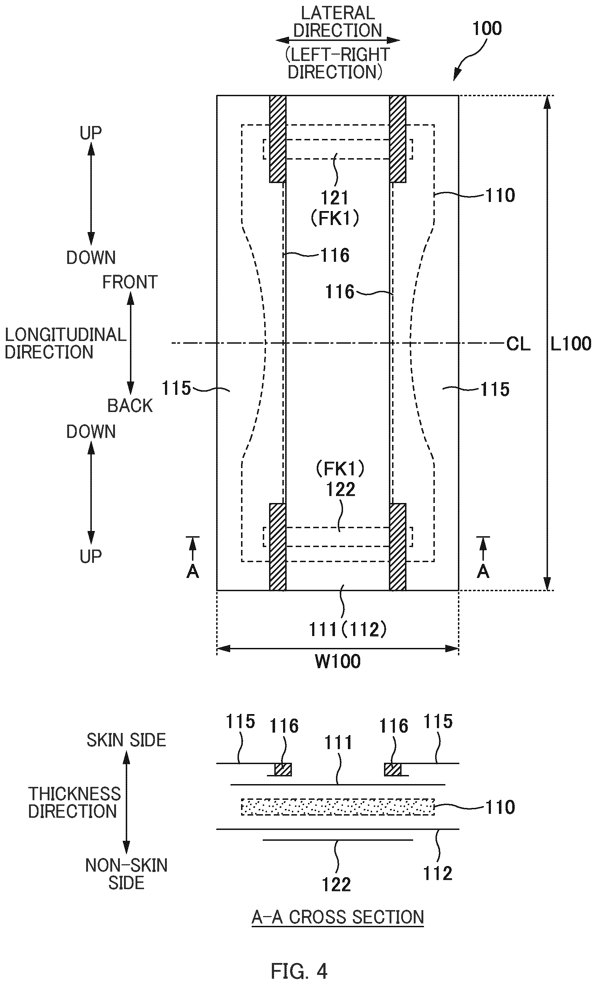

By engaging the engagement regions 30 provided on the wearable article 1 respectively with absorbent pad engagement regions 120 (see FIG. 4 described further below) provided on the absorbent pad 100, the absorbent pad 100 can be attached to and retained on the inner side (skin side) of the wearable article 1. Further, the engagement regions 30 function as a reference (i.e., a guide) for when a user attaches the absorbent pad 100 to the wearable article 1; by visually identifying the engagement regions 30, the user can attach the absorbent pad 100 in a proper position of the wearable article 1.

In the present embodiment, a hook member FK is provided in the engagement region 30. The hook member FK is either one of: a male hook member FK1 in which a plurality of engagement protrusions are provided on the surface of a base sheet; and female hook member FK2 in which a plurality of engagement loops are provided on the surface of a base sheet. By bringing the male hook member FK1 and the female hook member FK2 in opposition to one another and engaging the engagement protrusions of the male hook member FK1 with the engagement loops of the female hook member FK2, the hook members can engage with one another. The following describes an example in which a female hook member FK2 is provided in the engagement region 30 of the wearable article 1. Note that, for example, known hook-and-loop fasteners can be used as the hook members FK (FK1, FK2). Further, in order to improve visual recognizability, it is more preferable that the color of the hook member FK is different from the color of the knitted fabric constituting the main body portion 10.

An overview of a method for manufacturing the wearable article 1 is described below. FIGS. 3A to 3C are diagrams illustrating a method for manufacturing the wearable article 1. FIG. 3 illustrates an example in which the wearable article 1 is formed by the aforementioned "knitted fabric".

First, as illustrated in FIG. 3A, a tubular knitted fabric 10A (so-called "circular knitting") is made by knitting a thread in rounds. At this time, the stretch-suppressing portions 20 (21 to 24) are also formed along the direction of the center axis of the tube-shaped circular knitted fabric 10A (corresponding to the up-down direction in FIG. 1). As described above, the stretch-suppressing portions 20 are formed by applying such processes as changing the knitting pattern or sewing-in a different thread. Note, however, that the stretch-suppressing portions 20 may be formed in a step separate from the step of forming the circular knitted fabric 10A (FIG. 3A). For example, after forming the circular knitted fabric 10A, a process may be performed to separately form the stretch-suppressing portions 20 (stretch-suppressing means).

Next, the circular knitted fabric 10A is cut at predetermined positions in the up-down direction (axial direction), to cut out the main body portion 10 constituting an individual wearable article 1 as illustrated in FIG. 3B. In this example, the circular knitted fabric 10A is cut at positions C1 and C2 in the up-down direction (see FIG. 3A). The cutting position C1 becomes the upper side of the main body portion 10 in the up-down direction, and the cutting position C2 becomes the lower side of the main body portion 10 in the up-down direction. Further, portions that become the leg openings LH are also cut out from the main body portion 10. In FIG. 3B, both lateral portions in the left-right direction are cut obliquely at the lower end portion of the tubular main body portion 10, to thereby form portions that become the pair of leg openings LH, LH.

Next, as illustrated in FIG. 3C, the upper end portion, in the up-down direction, of the tubular main body portion 10 is folded back, to thereby form the waist fold-back portion 11. Further, at the lower end portion, in the up-down direction, of the tubular main body portion 10, the front-side lower edge 12f and the back-side lower edge 12b are sewn together from the back and front, to thereby form the crotch sewn portion 12. In this way, the tubular main body portion 10 is formed into an underpants shape. Then, overcast stitching is performed along the peripheral edge of the respective leg openings LH that have been cut out, to thereby form the leg-opening overcast-stitched portions 13. Finally, the hook members FK (female hook members FK2) are attached to the inner-side (skin-side) face of the main body portion 10, to thereby form the engagement regions 30 (31, 32). In this way, the wearable article 1 illustrated in FIGS. 1 and 2 is formed. Note that the order in which the processes illustrated in FIG. 3C are performed may be changed.

### Stretchability of Wearable Article 1:

The wearable article 1 has been described above as having stretchability in the up-down direction and the left-right direction. More specifically, the wearable article is defined as having stretchability in cases where the following condition is satisfied when a sample piece cut out from the knitted fabric constituting the main body portion 10 is subjected to a tensile test. That is, the wearable article is defined as having stretchability in cases where the maximum value of an elongation rate when a 50 mm × 200 mm sample piece is pulled with a force of 5 N in each of the up-down direction and the left-right direction is 200% or greater.

The tensile test can be conducted as follows, for example. First, a rectangular sample piece being 220 mm long in the up-down direction and 50 mm long in the left-right direction is cut out from the main body portion 10 of the wearable article 1. Note that, of the 220 mm length, portions worth 10 mm on both ends in the up-down direction of the sample piece are margins to be clamped by the chucks of a tensile tester. Further, at the time of cutting out the sample piece, it is preferable not to include the stretch-suppressing portion 20 and the engagement region 30 (hook member FK). Next, 10 mmm each of both end portions, in the up-down direction, of the cut-out sample piece are respectively clamped with the chucks of a known tensile tester (for example, a universal material testing system from Instron). At this time, the tensile tester's chuck-to-chuck distance G₀ is set to 200 mm. From this state, the sample piece is pulled and stretched at a tensile rate of about 200 mm/min in the direction in which the chuck-to-chuck distance increases, and when the tensile force (magnitude of force) by the tensile test reaches 5 N, the chuck-to-chuck distance G₁ (mm) is measured. Then, a value (=G₁/G₀) of the length of the sample piece after being pulled to the length thereof before being pulled is calculated and recorded as the elongation rate. This test is repeated a plurality of times, to find the maximum value of the elongation rate (G₁/G₀). From this result, in cases where the maximum value of the elongation rate (G₁/G₀) is 200% or greater, then the fabric constituting the wearable article 1 (main body portion 10) is found as having stretchability in the up-down direction. Note that the dimensions of the clamping margins for the chucks can be varied as appropriate.

Similarly, a rectangular sample piece being 220 mm long in the left-right direction and 50 mm long in the up-down direction is cut out from the main body portion 10 of the wearable article 1, and a tensile test in the left-right direction is performed according to the aforementioned procedure. In cases where the elongation rate is 200% or greater, then the fabric constituting the wearable article 1 (main body portion 10) is found as having stretchability in the left-right direction. Note that, in cases of cutting out a sample piece that is long in the left-right direction from the wearable article 1, the stretch-suppressing portion 20 may be included. This is because the stretch-suppressing portion 20 primarily functions to reduce the stretchability in the up-down direction, and does not greatly affect the measurement of stretchability in the left-right direction.

### Basic Configuration of Absorbent Pad 100:

FIG. 4 shows a plan view and a cross-sectional view of an absorbent pad 100. The absorbent pad 100 has a substantially rectangular planar-view shape, and has a longitudinal direction, a width direction, and a thickness direction orthogonal to one another. The longitudinal direction is the direction along the up-down direction (and the front-back direction) of the wearable article 1 when the absorbent pad is attached to the wearable article 1. The lateral direction is the direction along the left-right direction of the wearable article 1 when the absorbent pad is attached to the wearable article 1. The front side in the longitudinal direction is the side located on the wearer's stomach side (the front side of the wearable article 1 in the front-back direction) when the absorbent pad 100 is attached to the wearable article 1. The back side in the longitudinal direction is the side located on the wearer's back side (the back side of the wearable article 1 in the front-back direction) when the absorbent pad 100 is attached to the wearable article 1.

The absorbent pad 100 includes: an absorbent body 110; a top sheet 111 disposed on the skin side of the absorbent body 110; a back sheet 112 disposed on the non-skin side of the absorbent body 110; and a pair of side sheets 115, 115 provided more toward the skin side than the top sheet 111 and respectively on both sides in the left-right direction. The absorbent pad also includes absorbent pad engagement regions 120 to be engaged with the engagement regions 30 of the wearable article 1.

The absorbent body 110 includes a polymeric absorbent agent (super absorbent polymer; also called "SAP") and a liquid-absorbent fiber such as pulp fiber, and in the present embodiment, has a substantially hourglass-like shape in which the central portion in the longitudinal direction is narrowed inwardly in the left-right direction, as illustrated by the broken lines in FIG. 4. The SAP-containing liquid-absorbent fiber may be covered by a liquid-permeable core-wrapping sheet (not illustrated). Further, the configuration of the absorbent body 110 is not limited to the above, and other examples may include a SAP sheet wherein a SAP layer is attached to a hydrophilic sheet, and an air-laid sheet wherein the liquid-absorbent fiber is shaped into a sheet by an air-laid method.

The top sheet 111 is a liquid-permeable sheet member disposed on the skin side of the absorbent body 110, and is a member that comes into direct contact with the wearer's skin and/or excretory opening during use of the absorbent pad 100. Therefore, it is preferable that the top sheet 111 is a sheet member that is as soft as possible and has a soft texture to the touch. An example of the sheet member constituting the top sheet 111 of the present embodiment may include an air-through nonwoven fabric etc. Further, a second sheet (not illustrated in FIG. 4) that has liquid permeability substantially like the top sheet 111 may be provided between the absorbent body 110 and the top sheet 111 in the thickness direction.

The back sheet 112 is a liquid-impermeable, air-permeable sheet member disposed more toward the non-skin side than the absorbent body 110 in the thickness direction of the absorbent pad 100. By providing the back sheet 112, excreted fluid, such as urine, that has been absorbed by the absorbent pad 100 is suppressed from permeating through to the non-skin side in the thickness direction. Thus, it is possible to suppress excreted fluid, such as urine, that has been absorbed by the absorbent pad 100 from permeating to the wearable article 1 on the non-skin side when the absorbent pad 100 is used by being attached to the skin side of the wearable article 1. An example of a sheet member constituting the back sheet 112 of the present embodiment may include a resin film made from polyethylene, polypropylene, etc.

The side sheets 115 are members constituting both lateral portions in the lateral direction on the skin-side face of the absorbent pad 100. During use of the absorbent pad 100, the side sheets 115 are also highly likely to come into direct contact with the wearer's skin and are therefore preferably constituted by a sheet member that is as soft as possible, like the top sheet 111. An example of the sheet member constituting the side sheet 115 of the present embodiment may include an air-through nonwoven fabric etc.

In an inner end portion, in the lateral direction, of each of the pair of side sheets 115, 115, an absorbent pad elastic member 116 such as an elastic string etc. is provided in a stretched state along the longitudinal direction.

The inner end, in the lateral direction, of each side sheet 115 is folded back toward the outer side in the lateral direction, as illustrated in a cross-sectional view taken along cross section A-A of FIG. 4, and the absorbent pad elastic member 116 is attached so as to be sandwiched between the folded-back part. During use of the absorbent pad 100, the stretchability exerted by the absorbent pad elastic members 116 causes the respective inner end portions of the pair of side sheets 115, 115 to rise up toward the skin side in the thickness direction, thereby enabling the side sheets to function as leak-proof walls (barrier cuffs) and thus suppress side leakage of urine etc.

The absorbent pad engagement regions 120 are provided on the non-skin-side face of the back sheet 112, and are parts that detachably engage respectively with the engagement regions 30 on the wearable article 1, and include: a front-side absorbent pad engagement region 121 disposed in a front-side end portion in the longitudinal direction; and a back-side absorbent pad engagement region 122 disposed in a back-side end portion in the longitudinal direction. The absorbent pad engagement region 120 is provided with a male hook member FK1 that engages with the female hook member FK2 provided in the engagement region 30 of the wearable article 1. For the same reason as that for the female hook member FK2 (engagement region 30), it is preferable that the color of the male hook member FK1 is different from the color of the back sheet 112 to enable the user to visually recognize the engagement position easily.

When the absorbent pad 100 is to be used by being attached to the wearable article 1, the absorbent pad 100 is folded in two at the central position CL, in the longitudinal direction, of the absorbent pad 100 in a manner that the absorbent pad's skin-side face is on the inside. The bifolded absorbent pad 100 is then inserted from the upper side of the waist opening BH of the wearable article 1, and the position is adjusted such that the central position CL of the absorbent pad 100 in the longitudinal direction is located at the lowermost end portion (position overlapping the crotch sewn portion 12) of the wearable article 1. In this state, the front-side absorbent pad engagement region 121 (male hook member FK1) is engaged with the front-side engagement region 31 (female hook member FK2) of the wearable article 1, and the back-side absorbent pad engagement region 122 (male hook member FK1) is engaged with the back-side engagement region 32 (female hook member FK2) of the wearable article 1. Note that, since the position, in the longitudinal direction (up-down direction), of each absorbent pad engagement region 120 is adjusted in advance such that it overlaps the engagement region 30 of the wearable article 1 in a state where the absorbent pad 100 has been inserted inside the wearable article 1, the male hook member FK1 and the female hook member FK2 can be engaged with one another easily. In this way, the absorbent pad 100 can be attached properly to the wearable article 1.

### Actions for Putting on Wearable Article 1 When Using Absorbent Pad 100 in Combination

Next, specific actions for putting on the wearable article 1 when the absorbent pad 100 is used in combination will be described.

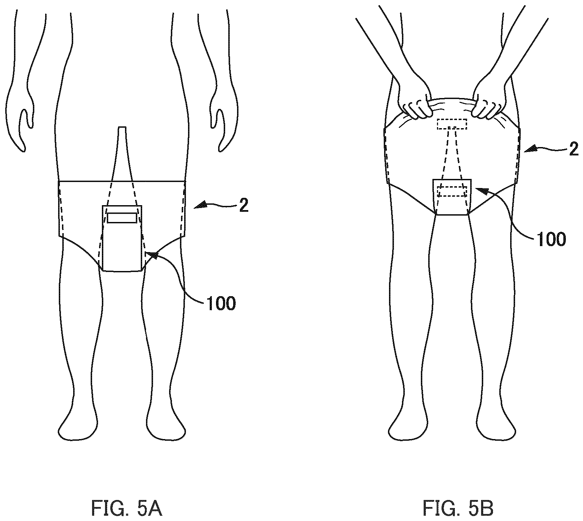

First, as a comparative example, actions for putting on a conventional underpants-shaped wearable article 2 (also referred to hereinafter as "wearable article 2"), while using an absorbent pad 100 in combination, will be described. FIGS. 5A and 5B are diagrams illustrating a method for putting on a wearable article 2 of a comparative example when the wearable article 2 is used in combination with an absorbent pad 100.

The wearable article 2 of the comparative example is a conventional underpants-shaped wearable article, is constituted by a fabric having stretchability as in the wearable article 1, and includes engagement regions each provided with a predetermined female hook member. As for the absorbent pad 100, it is possible to use an absorbent pad substantially identical to the absorbent pad 100 illustrated in FIG. 4, wherein the absorbent pad includes engagement regions each provided with a male hook member engageable with the female hook member on the wearable article 1 side.

In the comparative example, when the wearable article 2 is to be used in combination with the absorbent pad 100, first, the absorbent pad 100 is attached to the wearable article 2 as illustrated in FIG. 5A. That is, the female hook member of the wearable article 2 is brought into engagement with the male hook member of the absorbent pad 100. Then, in this state where the absorbent pad 100 has been attached, both legs of the wearer are passed respectively through the leg openings LH, LH of the wearable article 2, which is then pulled up to just above the knees. Note that the absorbent pad 100 may be attached after the wearer's legs have been inserted into the wearable article 2.

Next, as illustrated in FIG. 5B, an action is performed wherein the vicinity of the waist opening (the upper end portion of the waist region) of the wearable article 2 is gripped with the hands and the wearable article is pulled up to the wearer's crotch area. At this time, if the force with which the wearable article 2 is pulled upward is greater than the engagement force of the hook members FK (the male hook member and the female hook member) which engage the absorbent pad 100 and the wearable article 2, the engagement between the hook members may become disengaged. That is, a large shearing force may be exerted in the direction along the mutual engagement faces of the male hook member and the female hook member, and the engagement between the hook members may become disengaged. If the absorbent pad 100 and the wearable article 2 become disengaged, then it becomes difficult to apply tensile force to the absorbent pad 100, and it also becomes difficult to fit the wearable article to the wearer's crotch area.

Particularly, since the wearable article 2 is constituted by a highly stretchable fabric, if the hook members become disengaged, the wearable article 2 is likely to stretch greatly in the up-down direction as illustrated in FIG. 5B. In this case, in order to make the absorbent pad 100 fit the wearer's crotch area, it becomes necessary to pull the wearable article 2 with an even greater force or increase the pulling distance, which may cause the wearable article 2 to be stretched excessively, thus damaging or tearing the fabric. Further, in association with the stretching of the wearable article 2, the position of the absorbent pad 100 is likely to deviate, and thus, it may be difficult to make the absorbent pad 100 suitably fit the wearer's crotch area.

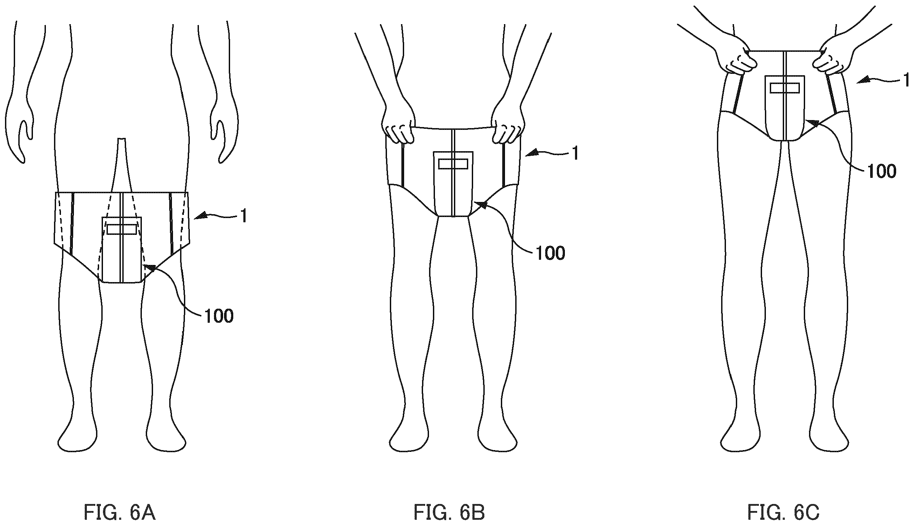

Next, actions for putting on the wearable article 1 of the present embodiment, while using an absorbent pad 100 in combination, will be described. FIGS. 6A to 6C are diagrams illustrating a method for putting on the wearable article 1 when the wearable article 1 is used in combination with an absorbent pad 100.

In the present embodiment, first, an absorbent pad 100 is attached to the wearable article 1. Attachment of the absorbent pad 100 is achieved by engaging the male hook member FK1 provided in the absorbent pad engagement region 120 of the absorbent pad 100 with the female hook member FK2 provided in the engagement region 30 of the wearable article 1, as described above. Then, as illustrated in FIG. 6A, in this state where the absorbent pad 100 has been attached, both legs of the wearer are passed respectively through the leg openings LH, LH of the wearable article 1, which is then pulled up to just above the knees.

Next, an action is performed wherein the wearable article 1 is pulled upward by gripping the waist region BR thereof. In FIG. 6B, the wearer is using both hands to pull the wearable article upward by gripping both lateral portions, in the left-right direction, of the waist region BR. In the present embodiment, hook members FK (male hook member FK1, female hook member FK2) having a sufficiently strong engagement force, compared to the wearable article 2 of the comparative example, are used to engage the absorbent pad 100 and the wearable article 1. More specifically, the absorbent pad 100 and the wearable article 1 are engaged with one another by using hook members FK designed such that the engagement between the male hook member FK1 and the female hook member FK2 does not become disengaged, even when the wearable article 1 is pulled up with a force causing the wearable article to be stretched from a natural state to twice the length in the up-down direction. Thus, both the wearable article 1 and the absorbent pad 100 can be pulled up without causing the latter to be disengaged from the former. Further, since the wearable article 1 is provided with the stretch-suppressing means (stretch-suppressing portions 20 etc.), the waist region BR is suppressed from stretching excessively, thus making it easy to pull up the absorbent pad 100 together with the wearable article 1.

After the wearable article 1 has been pulled up to the vicinity of the wearer's crotch area, the position of the wearable article 1 and the absorbent pad 100 is adjusted as illustrated in FIG. 6C. In the wearable article 1, the wearable article 1 and the absorbent pad 100 are firmly engaged via the hook members FK1, FK2, and thus, by adjusting the position of the wearable article 1 by pulling the same, the position of the absorbent pad 100 can be adjusted in conjunction therewith. Thus, the absorbent pad 100 can be fitted suitably to the wearer's crotch area.

As described above, in the present embodiment, the putting-on action can be performed smoothly by making the absorbent pad 100 engage with the wearable article 1 by using hook members FK whose engagement is less likely to become disengaged. Herein, the expression that the engagement of the hook members FK is "less likely to become disengaged" refers to the following state. Assume that F1 is the engagement force, per unit area, between the male hook member FK1 and the female hook member FK2 in a direction along mutual engagement faces of the hook members. Further, assume that F2 is the maximum tensile load, per unit area, for when a region located above the lower end of the engagement region 30 (female hook member FK2) up to 20 mm from the upper end of the wearable article 1 in the up-down direction of the waist region BR of the wearable article 1 is stretched from a natural state to a state (double-stretched state) in which the length of the region is stretched to twice the length in the up-down direction. In this case, the engagement is less likely to become disengaged when F1 is greater than F2.

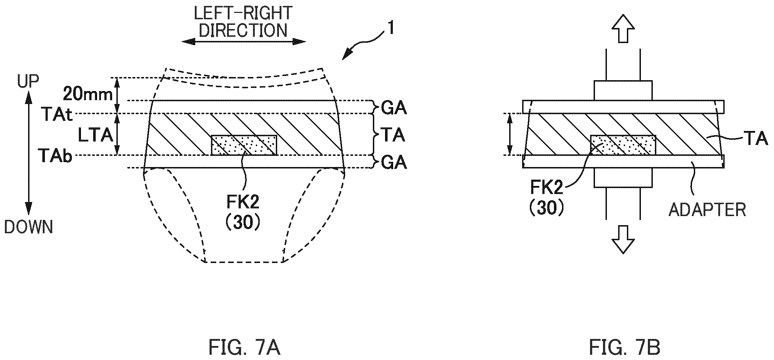

FIGS. 7A and 7B are diagrams illustrating a method for measuring the maximum tensile load at the time of stretching the wearable article 1 in the up-down direction. FIG. 7A illustrates a region to be subjected to a tensile test within the waist region BR of the wearable article 1. First, in either the front side (also referred to as "front panel") or the back side (also referred to as "back panel") in the front-back direction of the wearable article 1 in its natural state, a tensile region TA, shown in hatching in FIG. 7A, is set as a target region to be subjected to the tensile test. The tensile region TA is a portion within the waist region BR that is gripped and pulled with the hands during the putting-on action, and is a portion that includes the entire engagement region 30. More specifically, the tensile region is a portion having a length LTA in the up-down direction and being located above a lower end position TAb of the engagement region 30 up to a position TAt that is 20 mm below the upper end of the wearable article 1 in the up-down direction. The range of the tensile region TA is set below the position TAt because the portion where the wearer's fingers actually engage and apply force at the time of pulling up the wearable article 1, as illustrated in FIG. 6B, is at a position that is separated from the upper end of the wearable article 1 by at least 20 mm.

As illustrated in FIG. 7A, a portion including regions GA is cut out from the front panel of the wearable article 1 as a test piece, the regions GA each having a predetermined length in the up-down direction and being provided respectively in the upper and lower end portions of the tensile region TA shown in hatching. Each region GA is a portion serving as a margin to be clamped by a chuck of a tensile tester. Also, in cases of cutting out a test piece from the back panel side, a portion can be cut out so as to include the tensile region TA, which is located between the position TAt that is 20 mm below the upper end of the wearable article 1 and the lower end position TAb of the engagement region 30, and the regions GA serving as upper and lower clamping margins, in the same manner as the front panel.

The cut-out test piece (tensile region TA) is subjected to a tensile test in the up-down direction. FIG. 7B is a diagram illustrating a case in which the test piece is pulled in the up-down direction. The tensile test can be conducted by using a known tensile tester (for example, a universal material testing system from Instron) as described above. At the time of conducting the tensile test in the up-down direction, adapters or the like are attached to the chucks of the tensile tester so that the entire region in the left-right direction of the test piece can be clamped, and in this state, the regions GA (clamping margins) of the test piece are clamped from both above and below. Then the chuck-to-chuck distance G₀ is adjusted to length LTA of the tensile region TA in the up-down direction (G₀=LTA) in a manner that no creases are formed in the test piece. From this state, the test piece is stretched toward above and below at a rate of about 200 mm/min in the direction in which the chuck-to-chuck distance increases, and the maximum tensile load (N) detected when the chuck-to-chuck distance G₁ reaches 2LTA is recorded. This test is repeated a plurality of times, to find the average value of the maximum tensile load (N). In this way, it is possible to find the maximum tensile load for when the tensile region TA is stretched from a natural state to a state stretched to twice the length in the up-down direction (double-stretched state). By dividing the maximum tensile load by the area of the tensile region TA, it is possible to find the maximum tensile load per unit area (corresponding to the aforementioned F2) from a natural state to a state stretched to twice the length in the up-down direction.

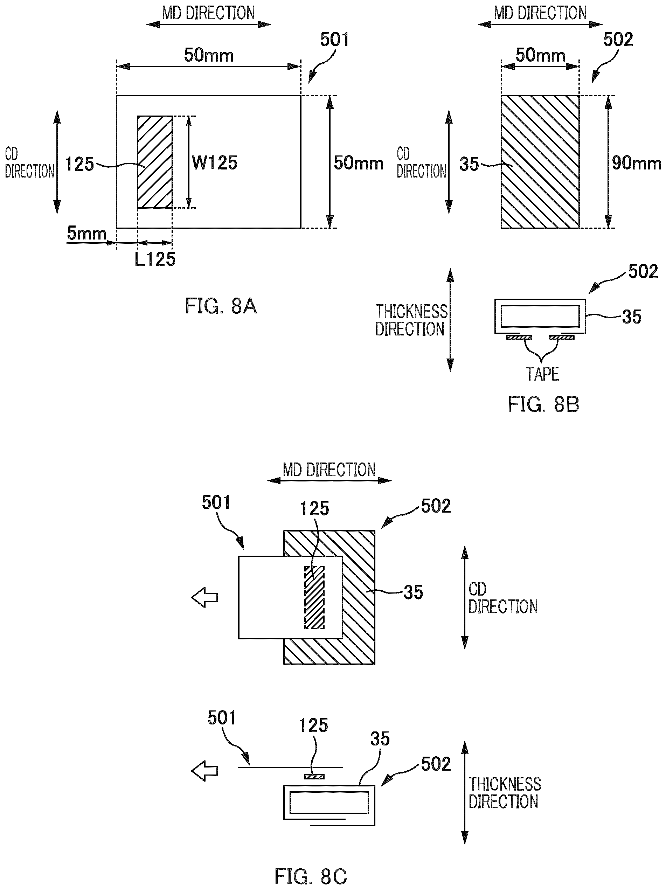

FIGS. 8A to 8C are diagrams illustrating a method for measuring the engagement force between a male hook member and a female hook member. Measurement of the engagement force is performed by preparing a sample piece 501 of a male hook member and a sample piece 502 of a female hook member, bringing the two sample pieces into engagement with one another, and measuring the maximum load until the engagement therebetween becomes disengaged when the sample pieces are pulled in opposite directions along their engagement faces.

FIG. 8A is a diagram illustrating the sample piece 501 of the male hook member used for the engagement force measurement. The sample piece 501 is made by attaching a male hook member 125 to be measured to a base sheet having been cut into a predetermined size. The base sheet is constituted by a release film or nonwoven fabric, and is 50 mm in the MD direction, which is the direction along the up-down direction of the wearable article 1, and 50 mm in the CD direction, which is the direction along the left-right direction of the wearable article 1. Note, however, that the size of the base sheet may be changed as appropriate. The length, in the MD direction, of the male hook member 125 to be attached to the base sheet is defined as L125, and the length thereof in the CD direction is defined as W125. The male hook member 125 is attached in an undetachable manner to a position separated by 5 mm from an end of the base sheet on one side (the left side in FIG. 8A) in the MD direction.

FIG. 8B is a diagram illustrating the sample piece 502 of the female hook member used for the engagement force measurement. The sample piece 502 is made by wrapping and fixing a female hook member 35 to be measured to a rectangular-parallelepiped substrate having a predetermined size. The substrate is an acrylic plate that is 50 mm in the MD direction and 90 mm in the CD direction. The female hook member 35 is wrapped along the MD direction of the substrate, and is fixed with a tape to one face in the thickness direction. Note, however, that the size of the substrate may be changed as appropriate depending on the size of the sample piece 501.

After the sample pieces 501, 502 have been prepared, the male hook member 125 of the sample piece 501 is arranged in opposition, in the thickness direction, to the female hook member 35 of the sample piece 502 and is engaged therewith as illustrated in FIG. 8C. At the time of engaging the hook members, they are lightly opposed such that the MD direction of the respective hook members is parallel to one another. Then, a pressure roller is moved back and forth once in the CD direction while applying a load of 2 kg in the thickness direction, to achieve a state wherein the male hook member 125 and the female hook member 35 are firmly engaged.

Next, both end portions, in the MD direction, of the sample pieces 501, 502 in an engaged state are respectively clamped with the chucks of a tensile tester. At this time, the chuck-to-chuck distance in the MD direction is set to 100 mm. Then, tension is applied toward both sides in the MD direction under conditions of a 5-kg load cell at a tensile rate of 100 mm/min, to measure the maximum tensile load until the engagement between the male hook member 125 and the female hook member 35 becomes disengaged. This measurement is repeated n times (e.g., five times), and the average value thereof is found as the maximum tensile load value. The engagement force, per unit area, between the male hook member 125 and the female hook member 35 (corresponding to F1 described above) is found by dividing the calculated maximum tensile load value by the area of the male hook member 125 (L35 × W35).

When the female hook member 35 is used as the female hook member FK2 provided in the engagement region 30 of the wearable article 1 and the male hook member 125 is used as the male hook member FK1 provided in the absorbent pad engagement region 120 of the absorbent pad 100, the engagement between the hook members FK is less likely to become disengaged in cases where the aforementioned engagement force F1 per unit area is greater than the maximum tensile load F2, per unit area, for when the wearable article 1 is stretched from the natural state to the double-stretched state (FK1 > FK2). That is, during the putting-on action of the wearable article 1, if the force, per unit area, in the direction along the engagement faces of the male hook member FK1 and the female hook member FK2 is stronger than the magnitude of the force, per unit area, when a predetermined region (tensile region TA) within the waist region BR of the wearable article 1 is pulled in the up-down direction, the engagement between the hook members FK is likely to be easily maintained against the force pulling the wearable article 1. Thus, when the wearable article 1 is pulled up in a state where the absorbent pad 100 is attached thereto, the engagement between the absorbent pad 100 and the wearable article 1 can be made less likely to disengage.

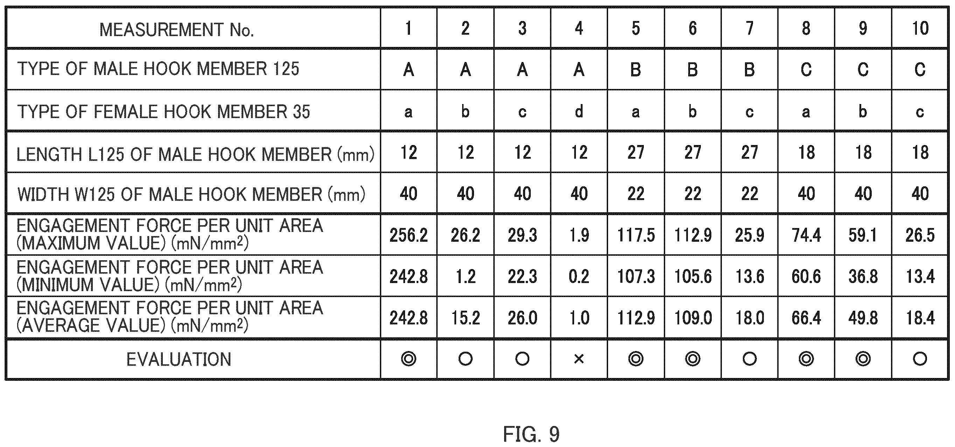

FIG. 9 is a table showing the results and evaluations for when the engagement force measurement illustrated in FIG. 8 was performed using a plurality of types of hook members. The engagement force of hook members was measured by using four types of hook members, A to D, as male hook members 125 and three types of hook members, a to c, as female hook members 35, and measurements were performed for ten combinations shown as Measurement Nos. 1 to 10. Further, the engagement force was evaluated for each combination. As for the engagement force evaluation, a plurality of (for example, ten) subjects evaluated how difficult it was to disengage the mutually-engaged male hook member 125 and female hook member 35 by pulling them in the direction (shearing direction) along their engagement faces. Cases in which none of the subjects were able to disengage the engagement between the hook members when each subject pulled the mutually-engaged hook members in the shearing direction were rated as "⊚", meaning that the engagement was extremely difficult to disengage. Cases in which at least 70% of the subjects could not disengage the engagement were rated as "∘", meaning that the engagement was difficult to disengage. Cases in which all of the subjects were able to disengage the engagement were rated as "×", meaning that the engagement was easy to disengage.

FIG. 9 shows that the combination of hook members in Measurement No. 4 was rated as "×" when the engagement force per unit area (average value) was 1.0 mN/mm², whereas the combinations of hook members in the other measurements were rated as "∘" or "⊚". It was thus verified that, when the average value of the engagement force when the male hook member and the female hook member were mutually engaged was at least equal to or greater than 15.2 mN/mm² (the average value in Measurement No. 2), the engagement between the hook members was difficult to disengage. The results also show that, from a safer viewpoint, when the minimum value of the engagement force is equal to or greater than 13.4 mN/mm² (the minimum value in Measurement No. 10), the rating is "o", indicating that the engagement between the hook members is difficult to disengage. Note that, in Measurement No. 2, the minimum value of the engagement force per unit area is measured to be 1.2 mN/mm², but this value greatly deviates from the other measurement results and there is a high possibility that this is a measurement error, and therefore, this data is not taken into consideration.

The above results show that, when the male hook member FK1 provided on the absorbent pad 100 and the female hook member FK2 provided on the wearable article 1 are in engagement, it is preferable that the engagement force, per unit area, in a direction along their mutual engagement faces is 13 mN/mm² or greater (a value on the safer side than 13.4 mN/mm², which is the minimum value in Measurement No. 10). By using hook members FK satisfying this condition, when the wearable article 1, with the absorbent pad 100 engaged therewith, is pulled up during the putting-on action of the wearable article 1, the engagement between the absorbent pad 100 and the wearable article 1 can be made less likely to disengage, compared to the opposite case (case in which the engagement force is less than 13 mN/mm²).

It has also been verified that cases in which the average value of the engagement force when the male hook member and the female hook member are in engagement is at least equal to or greater than 49.8 mN/mm² (the average value in Measurement No. 9) are rated as "⊚", indicating that the engagement between the hook members is more difficult to disengage. The results also show that, from a safer viewpoint, when the minimum value of the engagement force is equal to or greater than 36.8 mN/mm² (the minimum value in Measurement No. 2), the engagement between the hook members is difficult to disengage.

Therefore, when the male hook member FK1 provided on the absorbent pad 100 and the female hook member FK2 provided on the wearable article 1 are in engagement, it is more preferable that the engagement force, per unit area, in a direction along their mutual engagement faces is 35 mN/mm² or greater (a value on the safer side than 36.8 mN/mm², which is the minimum value in Measurement No. 2). By using hook members FK satisfying this condition, the engagement between the absorbent pad 100 and the wearable article 1 can be made even less likely to disengage, compared to the opposite case (case in which the engagement force is less than 35 mN/mm²).

Note that the wearable article 1 (main body portion 10) is constituted by a fabric whose maximum value of an elongation rate when a 50 mm × 200 mm sample piece is pulled at 5 N in the up-down direction or the left-right direction is 200% or greater in either direction. Using such a highly stretchable fabric improves the fit when the article is worn, and can also improve size conformability because the article can easily conform to the wearer's body size. Further, even when the wearable article 1 is stretched in the up-down direction during the putting-on action by using such a highly stretchable fabric, the engagement between the absorbent pad 100 and the wearable article 1 can be made less likely to disengage by using hook members FK that satisfy the aforementioned engagement force conditions.

Further, of the male hook member FK1 and the female hook member FK2, the female hook member FK2 is provided in the engagement region 30 of the wearable article 1 of the present embodiment. When the wearable article 1 is worn, the engagement region 30 is located on the wearer's skin side and may come into contact with the wearer's skin. Therefore, if the male hook member FK1 is provided in the engagement region 30, the engagement protrusions of the male hook member FK1 may damage the wearer's skin. In contrast, the female hook member FK2 provided in the engagement region 30 of the wearable article 1 does not have engagement protrusions and is softer than the male hook member FK1. Therefore, the female hook member is less likely to damage the wearer's skin, even when it comes into contact with the wearer's skin.

Further, as described above, the wearable article 1 can be washed and used repeatedly, and in this case, since the female hook member FK2 does not have engagement protrusions, the knitted fabric constituting the main body portion 10 of the wearable article 1 is suppressed from being damaged by the engagement protrusions at the time of washing. Further, since the female hook member FK2 is soft, the wearable article 1 can be folded easily after being washed, and it is thus suitable for wearable articles 1 that can be used repeatedly.

At this time, it is preferable that the female hook member FK2 is sewn onto the skin-side face of the wearable article 1 with a thread etc. By being sewn on with a thread etc., the female hook member FK2 is undetachably fixed, and thus, the female hook member FK2 itself can be suppressed from peeling off from the engagement region 30 of the wearable article 1. Further, since the female hook member FK2 is less likely to peel off even when the wearable article 1 is washed, the user can wash the wearable article without concern. Further, since joining with the wearable article 1 does not employ an adhesive etc., the female hook member is safe to the human body and can easily offer a soft texture, even when it comes into contact with the wearer's skin on the skin-side face of the wearable article 1.

Further, in the wearable article 1, the hook member FK (engagement region 30) is disposed in the waist region BR (see FIG. 2). If the hook member FK were disposed in the crotch region CR below the waist region BR, the distance from the upper end of the wearable article 1 will become large, which will make it difficult for the force for pulling up the waist region BR to be conveyed to the hook member FK during the putting-on action of the wearable article 1, thus making it difficult to pull up the absorbent pad 100. In contrast, in the wearable article 1 of the present embodiment, since the hook member FK is disposed in the waist region BR, the distance from the upper end of the wearable article 1 is shorter compared to the aforementioned case, and thus, the force for pulling up the waist region BR is conveyed efficiently to the hook member FK. Thus, the absorbent pad 100 can be pulled upward more easily.

Further, in the up-down direction, the central position CFK of the hook member FK (engagement region 30) is located below the central position CLBR of the waist region BR (see FIG. 2). There is a high possibility that the region above the central position CLBR of the waist region BR (i.e., the upper-half region of the waist region BR) will be gripped directly with the hands during the putting-on action of the wearable article 1. Therefore, if the hook member FK is disposed in the upper-half region of the waist region BR and the absorbent pad 100 is in engagement therewith, then the female hook member FK2 and the female hook member FK2 may be gripped with the hands and thus the engagement therebetween may be peeled apart. In contrast, in the wearable article 1 of the present embodiment, the central position CFK of the hook member FK is disposed below the central position CLBR of the waist region BR, and thus, there is a low possibility that the hook member FK will be gripped with the hands during the putting-on action. Thus, the engagement between the male hook member FK1 and the female hook member FK2 can be made less likely to peel apart.

Further, the waist fold-back portion 11 is provided at the peripheral edge of the waist opening BH of the wearable article 1. This waist fold-back portion 11 has a strong contractive force as a result of, for example, knitting-in a thread that is less prone to extension/contraction. Stated differently, in the waist fold-back portion, the magnitude of force required for stretching the same by a unit length along a peripheral edge portion of the waist opening BH is greater than the magnitude of force required for stretching other regions by the unit length. That is, the wearable article 1 has a waist low-stretch region, along the peripheral edge of the waist opening BH, that is less prone to extension/contraction than other regions of the waist region BR.

Further, in the up-down direction, the hook member FK is disposed below the waist low-stretch region (waist fold-back portion 11) (see FIG. 2). Stated differently, the hook member FK is disposed in a position separated by a predetermined distance toward the lower side from the waist low-stretch region (waist fold-back portion 11). In a state where the wearer is wearing the wearable article 1, the waist low-stretch region (waist fold-back portion 11) exerts a stronger contractive force compared to other regions. Therefore, if the absorbent pad 100 were engaged in a position overlapping the waist low-stretch region (waist fold-back portion 11), then the absorbent pad 100 and the male hook member FK1 might be pressed firmly against the wearer's stomach or waist at the peripheral edge of the waist opening BH and might cause an uncomfortable feel or unnatural feel to the wearer. In contrast, disposing the hook member FK in a position separated from the waist low-stretch region (waist fold-back portion 11) helps to suppress the absorbent pad 100, which is engaged via the hook member FK, from being pressed against the wearer's skin side. Thus, it is possible to inhibit causing an uncomfortable feel to the wearer when the wearable article 1 is worn.

More specifically, when the length (width) of the waist low-stretch region (waist fold-back portion 11) in the up-down direction is defined as W11, the hook member FK is disposed below the lower end of the waist low-stretch region (waist fold-back portion 11) in the up-down direction by a distance of W11 or greater (see FIG. 2A). By disposing the hook member FK in a position separated by at least the width W11 of the waist fold-back portion 11, the hook member FK is less likely to be affected by the extension/contraction of the waist fold-back portion 11, compared to the opposite case. This helps to further suppress the absorbent pad 100, which is engaged via the hook member FK, from being pressed against the wearer's skin side, and thus it is possible to inhibit causing an uncomfortable feel to the wearer.

Further, it is more preferable that, in the up-down direction of the wearable article 1, the central position CFK of the hook member FK (engagement region 30) is located below the central position CLBR of the waist region BR and above the central position QLBR between the central position CLBR of the waist region BR and the lower end position (position LHt) of the waist region BR (see FIG. 2). Stated differently, it is preferable that, when the waist region BR is divided into four equal parts, the central position CFK of the hook member FK is located in the third region from the top in the up-down direction. With this arrangement, the distance from the upper end of the wearable article 1 is closer compared to the case where the central position CFK of the hook member FK is located in the fourth region from the top when the waist region BR is divided into four equal parts, and thus, the force for pulling the waist region BR upward is conveyed easily to the hook member FK. Further, the distance from the waist low-stretch region (waist fold-back portion 11) is farther compared to the case where the hook member's central position is located in the second region from the top when the waist region BR is divided into four equal parts, and thus, the influence caused by the strong contractive force in the waist low-stretch region is suppressed. That is, it is possible to pull up the absorbent pad 100 easily because the force for pulling up the waist region BR is conveyed to the hook member FK, and also, it is possible to inhibit causing an uncomfortable feel to the wearer because the strong contractive force along the peripheral edge of the waist opening BH (the waist low-stretch region) is prevented from acting excessively.

Further, in the wearable article 1 in a natural state, the upper end LHt of each leg opening LH is located above the lower end of the wearable article 1 in the up-down direction (in FIG. 2A, the same level as the lower end LHb of the leg opening LH) at a central position in the left-right direction. That is, the wearable article 1 has a so-called underpants-like shape wherein the leg openings LH are cut obliquely upward from the inner side toward the outer side in the left-right direction and from the lower side toward the upper side in the up-down direction. For example, in cases where the wearable article has a so-called boxer-shorts shape wherein the leg openings are formed horizontally, the leg openings may likely be caught on the wearer's legs when the wearer's legs are passed through the leg openings and the wearable article is pulled upward during the putting-on action. In this case, the leg openings are less likely to move upward and the wearable article is likely to be stretched in the up-down direction, which may cause a large shearing force to be exerted on the engagement portion with the absorbent pad, making the engagement of the hook members prone to becoming disengaged.

In contrast, with an underpants-like shape as in the wearable article 1 of the present embodiment, the leg openings LH are less likely to get caught on the wearer's legs, compared to a boxer-shorts-shaped wearable article, when the wearer's legs are passed through the leg openings LH and the wearable article is pulled upward during the putting-on action, thus making the entire wearable article 1 easy to pull up. Thus, the waist region BR is suppressed from being stretched excessively in the up-down direction when the waist region BR is gripped and pulled up, thereby reducing the shearing force exerted on the engagement portion with the absorbent pad 100. In this way, the engagement between the absorbent pad 100 and the wearable article 1 can be made less likely to disengage.

Further, in the front side (front panel side), in the front-back direction, of the wearable article 1 in a natural state, the peripheral edge of each leg opening LH has a shape that is narrowed inwardly in the left-right direction. That is, as illustrated in FIG. 2A, when the wearable article 1 in a natural state is viewed from the front side, the leg opening LH is formed in an arc shape that is convex toward the inner side in the left-right direction. By inwardly narrowing the peripheral edge of the leg opening LH, when the wearer moves his/her leg toward the front while wearing the wearable article 1, the peripheral edge of the leg opening LH is less likely to interfere with the wearer's leg, and it is possible to suppress causing an unnatural feel while the wearable article is worn. Further, during the putting-on action of the wearable article 1, the leg openings LH are less likely to get caught on the wearer's legs when the wearable article is pulled upward. Thus, the waist region BR is suppressed from being stretched in the up-down direction, thereby reducing the shearing force exerted on the engagement portion with the absorbent pad 100. In this way, the engagement between the absorbent pad 100 and the wearable article 1 can be made less likely to disengage.

Further, in the wearable article 1 of the present embodiment, in a natural state, the length (width) WCR of the crotch region CR in the left-right direction is from 25% to 50% of the length L0 of the wearable article 1 in the left-right direction (0.25 × L0 ≤ WCR ≤ 0.5 × L0) (see FIG. 2A). As described above, the knitted fabric constituting the wearable article 1 (main body portion 10) can be stretched by at least 200% in the left-right direction. The width of the leg opening LH in the left-right direction when stretched by 200% in the left-right direction (WLH; not illustrated) is expressed as WLH = (L - WCR)/2 = (2 × L0 - WCR)/2. Therefore, when the condition 0.25 × L0 ≤ WCR ≤ 0.5 × L0 is satisfied, the width of the leg opening LH in the left-right direction is 0.5 × L0 ≤ WLH ≤ 0.75 × L0.

During the putting-on action of the wearable article 1, in order to make it less likely for the wearer's legs to get caught on the peripheral edge of the leg openings LH at the time the wearer passes his/her legs through the leg openings LH, it is desirable that the leg opening LH is wider than the diameter of the wearer's leg. Typically, the width of the wearer's leg is equal to or less than 50% of the width of the wearer's waist. Therefore, by satisfying the aforementioned condition (0.5 × L0 ≤ WLH ≤ 0.75 × L0), the width WLH of the leg opening LH in the left-right direction when stretched during the putting-on action becomes wider than 50% of the width L0 of the wearer's waist. Thus, during the putting-on action of the wearable article 1, the leg opening LH can easily be made wider than the wearer's leg, and thus the leg can be passed through the leg opening LH easily. In this way, the wearable article 1 can easily be pulled up smoothly during the putting-on action, thus suppressing excessive stretching of the wearable article 1 in the up-down direction. Thus, force (shearing force) in the direction along the hook members' engagement faces is less likely to be exerted on the engagement faces of the hook members FK that engage the wearable article 1 and the absorbent pad 100. Further, the width WCR of the crotch region CR in the left-right direction can be provided with a certain breadth with respect to the width L0 of the entire wearable article 1 (0.25 × L0 ≤ WCRWLH ≤ 0.75 × L0), and thus, the absorbent pad 100 can easily be retained stably in the crotch region CR. With these configurations, the engagement between the absorbent pad 100 and the wearable article 1 can be made less likely to disengage.

Further, the wearable article 1 includes stretch-suppressing means (stretch-suppressing portions 20) each having a predetermined length in the up-down direction. In portions where the stretch-suppressing means are provided, the fabric constituting the main body portion 10 is less likely to stretch in the up-down direction when the wearable article 1 is pulled upward during the putting-on action. Thus, force (shearing force) in the direction along the hook members' engagement faces is less likely to be exerted on the engagement faces of the hook members FK that engage the wearable article 1 and the absorbent pad 100. In this way, the engagement between the absorbent pad 100 and the wearable article 1 can be made less likely to disengage.

Further, the stretch-suppressing means (stretch-suppressing portion 20) of the wearable article 1 has a portion overlapping the hook member FK (engagement region 30) in the up-down direction (see FIG. 2). With this configuration, in the portion in which the stretch-suppressing means (e.g., the front-side central stretch-suppressing portion 21) and the hook member FK (female hook member FK2) overlap one another, the fabric constituting the main body portion 10 is made less likely to stretch in the up-down direction. Thus, the shearing force exerted on the engagement faces of the hook members FK is further reduced. In this way, the engagement between the absorbent pad 100 and the wearable article 1 can be made even less likely to disengage.

Further, in the up-down direction, the stretch-suppressing means (stretch-suppressing portion 20) is disposed continuously at least from the waist low-stretch region (waist fold-back portion 11), which is provided along the peripheral edge of the waist opening BH, to the hook member FK (engagement region 30) (see FIG. 2). Stated differently, the waist low-stretch region (waist fold-back portion 11) and the hook member FK (engagement region 30) are vertically coupled by the stretch-suppressing means (stretch-suppressing portion 20). Thus, the mutually coupled waist low-stretch region (waist fold-back portion 11) and hook member FK (engagement region 30) function like a skeletal frame in the waist region BR of the wearable article 1 and help to suppress stretching in the up-down direction. That is, during the putting-on action of the wearable article 1, the region that is gripped and pulled up with the hands is less likely to stretch in the up-down direction. In this way, force in the direction along the engagement faces of the hook members FK is less likely to be exerted, and the engagement between the absorbent pad 100 and the wearable article 1 can be made even less likely to disengage.

Further, the stretch-suppressing means (stretch-suppressing portions 20) include: a central stretch-suppressing portion 21 (22) (central stretch-suppressing means) located in a central portion in the left-right direction and a pair of lateral stretch-suppressing portions 23, 23 (24, 24) (lateral-portion stretch-suppressing means) located respectively on both sides of the central stretch-suppressing portion 21 (22) in the left-right direction. During the putting-on action of the wearable article 1, an action is performed wherein both lateral portions of the waist region BR are gripped and pulled upward with the hands (see FIG. 6B). By providing the lateral stretch-suppressing portions 23 (24) in both lateral portions of the waist region BR, the portion gripped with the hands becomes less likely to stretch in the up-down direction. Further, since also the central stretch-suppressing portion 21 (22) is provided in the central portion in the left-right direction, the waist region BR, in its entirety, becomes less likely to stretch in the up-down direction. Thus, force in the direction along the hook members' engagement faces is less likely to be exerted on the engagement faces of the hook members FK that engage the wearable article 1 and the absorbent pad 100. In this way, the engagement between the absorbent pad 100 and the wearable article 1 can be made less likely to disengage.

Further, each lateral stretch-suppressing portion 23 (24, 24) has a portion intersecting with the leg-opening overcast-stitched portion 13 formed along the peripheral edge of each leg opening LH. With this configuration, during the putting-on action of the wearable article 1, the force applied by gripping and pulling up both lateral portions of the waist region BR can be conveyed easily to the peripheral edge (overcast-stitched portion 13) of the leg openings LH via the lateral stretch-suppressing portions 23 (24), and the leg openings LH can be pulled upward easily. That is, the leg openings LH are less likely to get caught on the wearer's legs, and thus, the wearable article 1 can be pulled up smoothly, making it less likely for the waist region BR to be stretched in the up-down direction. Thus, force (shearing force) in the direction along the hook members' engagement faces is less likely to be exerted on the engagement faces of the hook members FK that engage the wearable article 1 and the absorbent pad 100. In this way, the engagement between the absorbent pad 100 and the wearable article 1 can be made less likely to disengage.

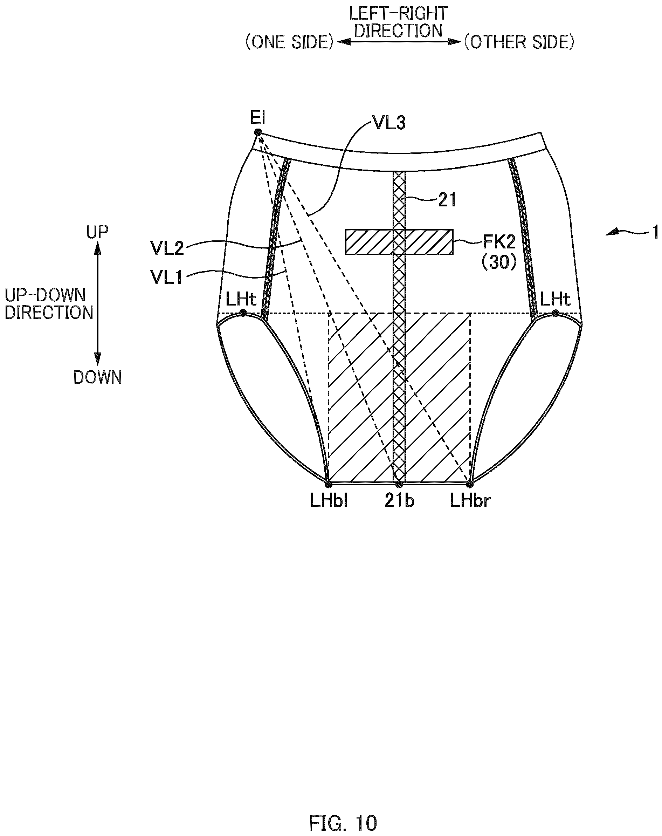

FIG. 10 is a diagram illustrating a configuration of the hook member FK (engagement region 30) and is a plan view illustrating a state in which the wearable article 1 in a natural state is viewed from the front side. In FIG. 10, an end of the wearable article 1 on the upper side in the up-down direction on one side (the left side in FIG. 10) in the left-right direction is defined as the force application point El. It has been described above that, during the putting-on action of the wearable article 1, both lateral portions of the waist region BR are gripped with the hands and pulled upward (see FIG. 6B etc.); at this time, It is assumed that the force application point El is gripped with the hand and force is applied thereto.

Further, in FIG. 10, the end located on the lower side in the up-down direction and on the inner side in the left-right direction of one leg opening LH, of the pair of leg openings LH, LH, located on one side (the left side) in the left-right direction is defined as one-side lower end LHbl. When the wearer's legs are passed through the leg openings LH and the wearable article 1 is pulled up, the one-side lower end LHbl comes into contact with and causes friction with the inner side of the wearer's leg (i.e., the vicinity of the inner thigh). Thus, in an action of pulling up the wearable article 1, tension occurs between the force application point El and the one-side lower end LHbl.

In the wearable article 1 of the present embodiment, when a straight line connecting the force application point El and the one-side lower end LHbl in a natural state is defined as a first imaginary line VL1, there is no overlapping portion between the hook member FK (engagement region 30) and the first imaginary line VL1. Stated differently, the hook member FK (engagement region 30) is provided in a position separated by a predetermined distance from the first imaginary line VL1. As described above, when the wearable article 1 is pulled up, tension occurs along the first imaginary line VL1. So, if the first imaginary line VL1 and the hook member FK (engagement region 30) overlap one another, force (shearing force) may be exerted in the direction along the engagement faces of the hook members FK, which may make it likely for the engagement between the male hook member FK1 and the female hook member FK2 to become disengaged. In contrast, in the wearable article 1, there is no portion in which the first imaginary line VL1 and the hook member FK (engagement region 30) overlap one another as illustrated in FIG. 10, and therefore, tension along the first imaginary line VL1 is suppressed from being exerted on the hook member FK. Thus, the engagement between the absorbent pad 100 and the wearable article 1 can be made less likely to disengage.

Further, in FIG. 10, the end of the stretch-suppressing means (central stretch-suppressing portion 21) on the lower side in the up-down direction is defined as central stretch-suppressing portion lower end 21b. When the wearer's legs are passed through the leg openings LH and the wearable article 1 is pulled up, the force applied to the force application point El is exerted on the lower end 21b of the central stretch-suppressing portion 21, and thereby, the wearable article 1 and the absorbent pad 100 are pulled upward. That is, in an action of pulling up the wearable article 1, tension occurs between the force application point El and the central stretch-suppressing portion lower end 21b.

In the wearable article 1, when a straight line connecting the force application point El and the central stretch-suppressing portion lower end 21b in a natural state is defined as a second imaginary line VL2, there is no overlapping portion between the hook member FK (engagement region 30) and the second imaginary line VL2. Stated differently, the hook member FK (engagement region 30) is provided in a position separated by a predetermined distance from the second imaginary line VL2. With this configuration, it is easy to suppress tension along the second imaginary line VL2 from being exerted on the hook member FK. Thus, the engagement between the absorbent pad 100 and the wearable article 1 can be made less likely to disengage.

Further, in FIG. 10, the end located on the lower side in the up-down direction and on the inner side in the left-right direction of one leg opening LH, of the pair of leg openings LH, LH, located on the other side (the right side) in the left-right direction is defined as other-side lower end LHbr. When the wearer's legs are passed through the leg openings LH and the wearable article 1 is pulled up, also the other-side lower end LHbr comes into contact with and causes friction with the inner side of the wearer's leg (i.e., the inner thigh), similar to the aforementioned one-side lower end LHbl. Thus, in an action of pulling up the wearable article 1, tension also occurs between the force application point El and the other-side lower end LHbr.

In the wearable article 1, when a straight line connecting the force application point El and the other-side lower end LHbr in a natural state is defined as a third imaginary line VL3, there is no overlapping portion between the hook member FK (engagement region 30) and the third imaginary line VL3. Stated differently, the hook member FK (engagement region 30) is provided in a position separated by a predetermined distance from the third imaginary line VL3. With this configuration, it is easy to suppress tension along the third imaginary line VL3 from being exerted on the hook member FK.

Thus, the engagement between the absorbent pad 100 and the wearable article 1 can be made less likely to disengage.

Further, of the hook members FK, the male hook member FK1 is provided in the absorbent pad engagement region 120 of the absorbent pad 100, as illustrated in FIG. 4. By providing the male hook member FK1, which easily engages with the female hook member FK2 provided in the engagement region 30 of the wearable article 1, the absorbent pad 100 can be engaged stably with the wearable article 1. Thus, even when the wearable article 1 is pulled upward with the absorbent pad 100 engaged therewith, the engagement is less likely to become disengaged, and the absorbent pad 100 can be made less likely to detach from the wearable article 1. Note that, in the absorbent pad 100, the male hook member FK1 (absorbent pad engagement region 120) is provided on the non-skin side in the thickness direction. Therefore, when the wearable article 1 is worn, there is a low possibility that the male hook member FK1 (absorbent pad engagement region 120), which has a higher rigidity than the female hook member FK2, will come into contact with the wearer's skin, thus inhibiting causing an uncomfortable feel to the wearer.

Further, when the absorbent pad 100 is viewed in the thickness direction, the hook member FK (absorbent pad engagement region 120) has a portion overlapping the absorbent body 110 (see FIG. 4). By overlapping the absorbent body 110 which has high rigidity, slack and deformation are less likely to occur on the surface of the hook member FK (male hook member FK1). That is, deformation of the hook member FK in the thickness direction is suppressed, and thus the hook member is easily maintained in a planar state. Thus, the hook member FK (male hook member FK1) of the absorbent pad 100 can be easily engaged with the hook member FK (female hook member FK2) provided in the engagement region 30 of the wearable article 1. Also, by being able to easily maintain the hook member FK (male hook member FK1) in a planar state, it is possible to suppress the engagement from coming off (peeling) due to curl-up of the end portion, in the up-down direction, of the hook member FK when the hook member, which is in engagement with the wearable article 1, is pulled upward. In this way, the engagement between the absorbent pad 100 and the wearable article 1 can be made less likely to disengage.

Further, in the absorbent pad 100, the hook member FK (male hook member FK1) is joined to the absorbent pad engagement region 120 by using an adhesive. At this time, the adhesive is applied to the entire surface of the hook member FK that comes into opposition with the absorbent pad engagement region 120. That is, the hook member FK provided on the absorbent pad 100 has no "dry edge", which is a portion where no adhesive is applied, along the peripheral edge of the hook member FK. If a dry edge were formed in the end portion of the hook member FK in the up-down direction, then disengagement (peeling) would likely occur at the dry edge, and the engagement with the wearable article 1 might become likely to disengage. In contrast, in the present absorbent pad 100, the adhesive is applied to the entire joining face of the hook member FK and no dry edge is formed, and thus, peeling etc. is suppressed. Thus, the engagement between the absorbent pad 100 and the wearable article 1 can be made less likely to disengage.

Note that the length L100 of the absorbent pad 100 in the longitudinal direction is preferably 300 mm or greater, and the length (width) W100 thereof in the lateral direction is preferably 80 mm or greater (L100 ≥ 300 mm; W100 ≥ 80 mm). When the length (width) W100 of the absorbent pad 100 in the lateral direction is 80 mm or greater, the width of the hook member FK (male hook member FK1) can be made wider compared to the opposite case (W100 < 80 mm), and thus, the hook member can engage more firmly with the wearable article 1.

Further, when the length L100 of the absorbent pad 100 in the longitudinal direction is 300 mm or greater, it is easier to secure a sufficient area for retaining SAP and liquid-absorbent fiber necessary for achieving water absorbency, compared to the opposite case (L100 < 300 mm). For example, assuming that a reference water absorption amount of the absorbent pad 100 is 220 cc, when the width W100 in the lateral direction is 80 mm, it is preferable that the length L100 in the longitudinal direction is 300 mm or greater in order to retain the necessary amount of SAP and liquid-absorbent fiber while keeping the thinness constant. With this length, it is easy to secure a sufficient area for covering up the wearer's genital area while maintaining water absorbency.

### Housing Body for Wearable Article 1:

Wearable articles 1 manufactured in a factory etc. are folded compactly and then shipped and distributed on the market in a state where one or more of the wearable articles are housed in a predetermined housing member. The following describes a wearable article housing body 90 in which a plurality of wearable articles 1 are housed in a housing member 70.

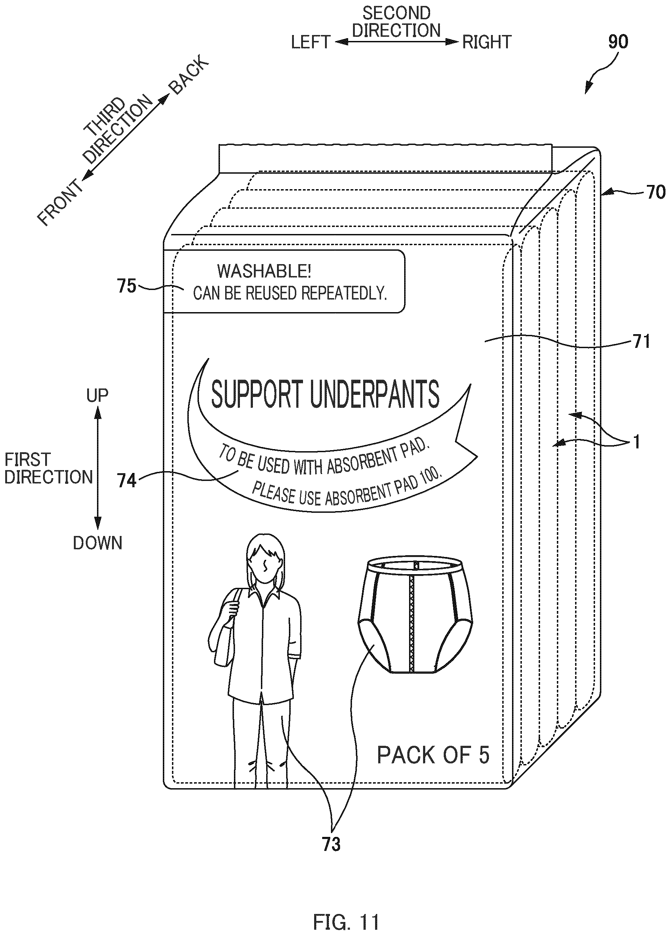

FIG. 11 is a schematic perspective view illustrating an example of a wearable article housing body 90 (also referred to hereinafter simply as "housing body 90"). The housing body 90 includes a housing member 70 which is a bag-shaped member, and one or more wearable articles 1 to be housed inside the housing member 70. Note that absorbent pads 100 are not housed in the housing body 90; when a user uses a wearable article 1 together with an absorbent pad 100, he/she needs to purchase or otherwise prepare the absorbent pad 100 separately.

As illustrated in FIG. 11, the housing body 90 has a first direction, a second direction, and a third direction intersecting with one another. One side of the first direction is the upper side, and the other side is the lower side.

Similarly, one side of the second direction is the right side, and the other side is the left side; and one side of the third direction is the front side, and the other side is the back side.

The housing member 70 is a substantially rectangular-parallelepiped container as illustrated in FIG. 11, and is formed by folding a base sheet 71, which is a sheet member constituting the housing member 70, and joining predetermined portions thereof. The base sheet 71 is a sheet member made from resin, and it is possible to use, for example, liquid-impermeable resin such as polyethylene, polypropylene, polyvinyl chloride, polystyrene, polycarbonate, polyethylene terephthalate (PET), etc. At the time of housing wearable articles 1 in the housing member 70, first, the base sheet 71 is folded and formed into a bag shape having an opening in the upper end portion in the first direction (not illustrated), and then a predetermined number of wearable articles 1 are housed through the upper end portion. After housing the wearable articles 1, the base sheet 71 is folded (in a gusset fold, for example) at the upper end portion as illustrated in FIG. 1, and then the opposing faces are joined together to close the opening. In this way, a substantially rectangular-parallelepiped housing body 90 is formed as illustrated in FIG. 11. Note, however, that the shape of the housing member 70, the method for folding the base sheet 71, and the method for enclosing the wearable articles 1 are not limited to the above.

The front-side face of the housing member 70 in the third direction corresponds to the frontal side of the housing body 90 which is viewed by a user in a store etc. Information, including letters and pictures, is indicated (printed) on the frontal face of the housing member 70. The example of FIG. 11 includes a product name indicating portion 72, a product image indicating portion 73, a description indicating portion 74, and an annotation indicating portion 75. The product name indicating portion 72 is a portion for indicating information showing the product name of the wearable article 1, and a user can use the product name indicating portion 72 as a guide to select and purchase the wearable article 1 from among a multitude of types of absorbent articles. The product image indicating portion 73 is a portion for indicating information showing an image related to the wearable article 1 and/or the user. The user looks at the product image indicating portion 73 and can thereby grasp, at a glance, an overview of the product and who the target wearer of the product is.

The description indicating portion 74 is a portion for indicating information showing a product description of the wearable article 1, and includes at least an indication which calls to mind that the wearable article is to be used in combination with the absorbent pad 100. In FIG. 11, there is an indication of information describing the fact that the wearable article 1 can be used in combination with an absorbent pad and the type of absorbent pad (absorbent pad 100) to be used in combination. The annotation indicating portion 75 is a portion for indicating an annotation other than the information indicated in the description indicating portion 74, and shows information that benefits the user. In FIG. 11, there is an indication of information describing that the wearable article 1 can be used repeatedly, and thereby, the user can recognize that the wearable article 1 is reusable.

Note that these information indicating portions 72-75 do not need to be printed on the base sheet 71 constituting the housing member 70, but may be formed, for example, by attaching stickers including indications of predetermined information onto the surface of the base sheet 71. Further, in cases where the base sheet 71 is a transparent sheet member, a card etc. having indications of predetermined information may be enclosed so that the card is visible from the outside.

By distributing such a housing body 90 on the market, a user having viewed the housing body 90 can easily recognize the functions of the wearable article 1 and how it is to be used, so that the wearable article 1 can be used properly.

### Washing of Wearable Article 1:

It has been described above that the wearable article 1 can be washed and used repeatedly, and in cases where the article is washed, it is important that the article is easy to dry. For example, in a case where it is assumed that the wearable article 1 is to be washed and dried while the wearer of the wearable article 1 is taking a bath, and the washed wearable article 1 is to be put on again after taking a bath, it is preferable that the wearable article 1 has high dryability.

In the wearable article 1 of the present embodiment, dryability is improved through the following configuration. First, a configuration for improving dryability will be described from the viewpoint of the fabric constituting the wearable article 1. In the present embodiment, the fabric constituting the wearable article 1 (main body portion 10) contains at least 90 wt% of polyester or polyethylene. Polyester and polyethylene are materials having a high degree of elongation compared to cotton, and thus, containing a large amount of such materials can further facilitate elongation. Further, polyester and polyethylene are materials having non-water absorbency and quick dryability, and thus, the time required for drying after washing can be reduced, leading to an improvement in the dryability of the wearable article 1. Note that the compositional makeup of the fabric (percentage by weight of each material) may be verified through measurement or may be verified by checking the "mixture ratio" written on a tag on the product.

Further, in the present embodiment, the fabric constituting the wearable article 1 does not contain any water-absorbent material. Examples of water-absorbent materials include cotton, rayon, etc. By not containing any water-absorbent material, the fabric is less likely to absorb water, and thus, the time required for drying after washing can be reduced, leading to an improvement in the dryability of the wearable article 1.

Further, the fabric constituting the wearable article 1 contains less than 10 wt% of a material having higher stretchability than polyester or polyethylene. Examples of materials having higher stretchability may include polyurethanes etc. Since the fabric constituting the wearable article 1 contains 90 wt% or more of polyester or polyethylene, by including the aforementioned material having higher stretchability in the remainder accounting for less than 10 wt%, it is possible to improve stretchability while securing good dryability. Note that, if the mixture ratio of the highly stretchable material is too high (10 wt% or more), then the fabric may shrink to a degree that creates creases in the wearable article 1 (main body portion 10) or the fabric may shrink excessively and make it likely for the legs to get easily caught at the time of passing the legs through the leg openings LH, LH. Therefore, it is preferable that the mixture ratio is less than 10 wt%.

Further, the fabric constituting the wearable article 1 includes a region that contains a material having higher stretchability than polyester or polyethylene and a region that does not contain the same. For example, by disposing the highly stretchable material in a region where fit is particularly needed in the wearable article 1 (i.e., by forming the region containing the material having higher stretchability), the fit can be further improved in a limited area of the wearable article 1, thereby improving functionality. Alternatively, in addition to improving the fit in a certain area, for example, by providing regions containing the material having higher stretchability in a plurality of areas with intervals therebetween, the fit of the wearable article 1 can be improved in its entirety.

Next, a configuration for improving dryability will be described from the viewpoint of the engagement region 30. As described above, it is preferable that the engagement region 30 is provided with a separate sheet member (e.g., a hook-and-loop fastener) that is different from the knitted fabric constituting the main body portion 10, and it is also preferable that the separate sheet member is constituted by polyethylene or polyester. Since the separate sheet member is joined to the main body portion 10, by making the separate sheet member from a material having quick dryability like the main body portion 10, the dryability of the wearable article 1 is improved.

Further, the separate sheet member is joined to the fabric constituting the wearable article 1 (main body portion 10) by being sewn with a thread along a peripheral edge portion of the separate sheet member. Thus, in a region on the inner side of the peripheral edge portion, which has been sewn with a thread, the fabric and the separate sheet member are not in tight adhesion (not joined) in the thickness direction, and there is a space formed between the separate sheet member and the fabric. That is, the entire separate sheet member is not in a state joined to the fabric, and therefore, compared to a case in which the entire sheet member is joined thereto, the face of the separate sheet member in contact with the fabric has a wide area in contact with air, which makes it easy for moisture to evaporate. Such a structure which allows air to escape easily improves dryability.

Further, in the present embodiment, when viewed in the thickness direction, there is a portion in which the separate sheet member and the stretch-suppressing portion 20 (the front-side central stretch-suppressing portion 21; the back-side central stretch-suppressing portion 22) overlap one another (see FIG. 2). More specifically, the separate sheet member is disposed so as to cross over a region (the stretch-suppressing portion 20) having a different contractive force. By providing the stretch-suppressing portion 20, a change in contractive force occurs in the fabric constituting the wearable article 1, and a space is more likely to be formed between the skin-side face of the fabric and the non-skin-side face of the separate sheet member. Dryability is improved by allowing air to easily escape from such a space.

Further, as regards the size of the separate sheet member, it is preferable that the length in the up-down direction is 50 mm or less and the length in the left-right direction is 150 mm or less. If the separate sheet member is too large, the sheet member portion may become difficult to dry. By adjusting the size within the aforementioned range, it becomes easier to dry while securing an area that can maintain sufficient engagement force with the fabric.

Further, it is preferable that the thickness of the separate sheet member is 3 mm or less. In this way, since the sheet member is not too thick, it is possible to maintain easy dryability while securing a thickness capable of maintaining sufficient engagement force with the fabric.

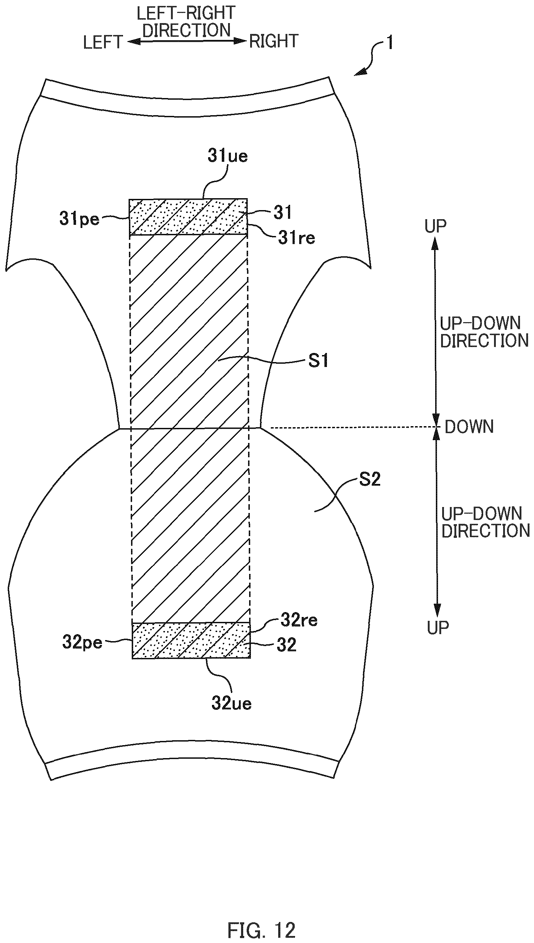

Next, a configuration for improving dryability will be described from another viewpoint. FIG. 12 is a schematic plan view illustrating a state in which both lateral ends, in the left-right direction, of the front-side waist region BR and back-side waist region BR of the wearable article 1 have been cut and the wearable article has been unfolded in the up-down direction. More specifically, the aforementioned "unfolded state" is a state in which the wearable article, which has been spread open in the up-down direction, is left to stand on a horizontal surface in a natural state. In the unfolded state of FIG. 12, the area of region S1 (the region shown in hatching in FIG. 12) is smaller than the area of the remaining region S2, the region S1 being a region between an upper end 31ue of the front-side engagement region 31 and an upper end 32ue of the back-side engagement region 32 in the up-down direction and between the respective ends 31pe, 32pe of the front-side engagement region 31 and back-side engagement region 32 most toward one side (the left side) and the respective ends 31re, 32re of the front-side engagement region 31 and back-side engagement region 32 most toward the other side (the right side) in the left-right direction. The region S1 within the fabric constituting the wearable article 1 is typically the region that is covered by the absorbent pad 100 when the absorbent pad 100 is attached. Therefore, if drying is somewhat insufficient after washing the wearable article 1, or if for some other reason the wearable article 1 gets wet after attaching the absorbent pad 100, the region S1 is likely to have low dryability thereafter because it is covered by the absorbent pad 100. However, by making the area of the region S1 smaller than the area of the region S2 in the fabric constituting the wearable article 1, it is easier to suppress deterioration of dryability of the entire wearable article 1, compared to the opposite case.

Note that, in the present embodiment, the area of the region S1 is smaller than the area of the remaining region S2, but this is not a limitation. Opposite from the above, the area of the region S1 may be larger than the area of the remaining region S2. In this case, even if drying is somewhat insufficient after washing the wearable article 1, the region S1 will be covered by the absorbent pad 100, and thus, the skin is less likely to become wet and wearing comfort is less likely to be impaired.

### OTHERS:

The foregoing embodiments are simply to facilitate understanding of the present invention and are not to be construed as limiting the present invention. It goes without saying that the present invention may be modified or altered without departing from its gist and encompasses equivalents thereof.

The foregoing embodiment describes an example in which stretch-suppressing portions 20 are provided as stretch-suppressing means configured to suppress stretching of the main body portion 10, but stretch-suppressing means other than the stretch-suppressing portions 20 may be provided. For example, providing the hook member FK in the engagement region 30 helps to suppress stretching of the main body portion 10 in the engagement region 30 (hook member FK). That is, the engagement region 30 (hook member FK) may also function as stretch-suppressing means.

### REFERENCE SIGNS LIST

1: Wearable article (underpants-shaped wearable article);
**2:** Wearable article (comparative example);
10: Main body portion;
11: Waist fold-back portion;
12: Crotch sewn portion;
13: Leg-opening overcast-stitched portion;
20: Stretch-suppressing portion (stretch-suppressing means);
21: Front-side central stretch-suppressing portion;
22: Back-side central stretch-suppressing portion;
23: Front-side lateral stretch-suppressing portion (lateral-portion stretch-suppressing means);
24: Back-side lateral stretch-suppressing portion (lateral-portion stretch-suppressing means);
30: Engagement region;
31: Front-side engagement region;
31pe: One-side end;
31re: Other-side end;
31ue: Upper end;
32: Back-side engagement region;
32pe: One-side end;
32re: Other-side end;
32ue: Upper end;
35: Female hook member;
70: Housing member;
71: Base sheet;
72: Product name indicating portion;
73: Product image indicating portion;
74: Description indicating portion;
75: Annotation indicating portion;
90: Wearable article housing body;
100: Absorbent pad;
110: Absorbent body;
111: Top sheet;
112: Back sheet;
115: Side sheet;
116: Absorbent pad elastic member;
120: Absorbent pad engagement region;
121: Front-side absorbent pad engagement region;
122: Back-side absorbent pad engagement region;
125: Male hook member;
501: Sample piece;
502: Sample piece;
BH: Waist opening;
LH: Leg opening;
LHt: Upper end;
LHb: Lower end;
BR: Waist region;
CR: Crotch region;
FK: Hook member;
FK1: Male hook member;
FK2: Female hook member;
VL1: First imaginary line;
VL2: Second imaginary line;
VL3: third imaginary line;
S1: Region;
S2: Region.

## Claims

1. A wearable article housing body comprising:
a wearable article having an up-down direction, a left-right direction, and a front-back direction intersecting with one another, the wearable article having stretchability in the up-down direction and the left-right direction and to be used in combination with an absorbent pad; and
a housing member configured to house the wearable article,
the wearable article being reusable,
the housing member having an indication that calls to mind that the wearable article is to be used in combination with the absorbent pad,
the wearable article comprising a hook member configured to engage with the absorbent pad, the hook member being either one of a male hook member and a female hook member,
a state in which the wearable article is taken out from the housing member and stretched in length in the up-down direction to twice the length from a natural state being defined as a double-stretched state,
an engagement force, per unit area, between the male hook member and the female hook member in a direction along mutual engagement faces of the male hook member and the female hook member being greater than a maximum tensile load, per unit area, for when a region located above a lower end of the hook member up to 20 mm from an upper end of the wearable article is stretched from the natural state to the double-stretched state.

2. A wearable article having an up-down direction, a left-right direction, and a front-back direction intersecting with one another, the wearable article having stretchability in the up-down direction and the left-right direction and to be used in combination with an absorbent pad,
the wearable article comprising:
a hook member configured to engage with the absorbent pad, the hook member being either one of a male hook member and a female hook member,
a state in which the wearable article is stretched in length in the up-down direction to twice the length from a natural state being defined as a double-stretched state,
an engagement force, per unit area, between the male hook member and the female hook member in a direction along mutual engagement faces of the male hook member and the female hook member being greater than a maximum tensile load, per unit area, for when a region located above a lower end of the hook member up to 20 mm from an upper end of the wearable article is stretched from the natural state to the double-stretched state.

3. The wearable article according to claim 2, wherein
the engagement force, per unit area, in the direction along the mutual engagement faces between the male hook member and the female hook member is 13 mN/mm² or greater.

4. The wearable article according to claim 3, wherein
the engagement force, per unit area, in the direction along the mutual engagement faces between the male hook member and the female hook member is 35 mN/mm² or greater.

5. The wearable article according to any one of claims 2 to 4, comprising
a main body portion constituted by a fabric whose maximum value of an elongation rate when a 50 mm × 200 mm sample piece is pulled at 5 N in the up-down direction or the left-right direction is 200% or greater.

6. The wearable article according to any one of claims 2 to 4, wherein
the hook member is the female hook member.

7. The wearable article according to claim 6, wherein
the female hook member is sewn onto a skin-side face of the wearable article.

8. The wearable article according to any one of claims 2 to 4, comprising:
a pair of leg openings; and
a waist region located between an upper end of each of the leg openings and the upper end of the wearable article in the up-down direction, wherein
the hook member is disposed in the waist region.

9. The wearable article according to claim 8, wherein
in the up-down direction, a central position of the hook member is located below a central position of the waist region.

10. The wearable article according to claim 8, comprising:
a waist opening; and
a waist low-stretch region in which a magnitude of force required for stretching the waist low-stretch region by a unit length along a peripheral edge portion of the waist opening is greater than a magnitude of force required for stretching another region by the unit length, wherein
in the up-down direction, the hook member is disposed below the waist low-stretch region.

11. The wearable article according to claim 10, wherein
in the up-down direction, the hook member is disposed below a lower end of the waist low-stretch region by a distance greater than or equal to a width of the waist low-stretch region in the up-down direction.

12. The wearable article according to claim 8, wherein
in the up-down direction, a central position of the hook member is located above a central position between a central position of the waist region and a lower end of the waist region.

13. The wearable article according to any one of claims 2 to 4, comprising
a pair of leg openings, wherein
in the natural state, an upper end of each of the leg openings is located above a lower end of the wearable article in the up-down direction at a central position in the left-right direction.

14. The wearable article according to claim 13, wherein
in the natural state, a peripheral edge of each of the leg openings in a front side in the front-back direction has a shape that is narrowed inwardly in the left-right direction.

15. The wearable article according to claim 14, comprising
a crotch region located between an upper end of each of the leg openings and a lower end of the wearable article in the up-down direction and between respective inner ends of the pair of leg openings in the left-right direction, wherein
a length, in the left-right direction, of the crotch region in the natural state is from 25% to 50% of a length, in the left-right direction, of the wearable article in the natural state.

16. The wearable article according to any one of claims 2 to 4, comprising
stretch-suppressing means having a predetermined length in the up-down direction.

17. The wearable article according to claim 16, wherein
in the up-down direction, there is a portion in which the stretch-suppressing means and the hook member overlap one another.

18. The wearable article according to claim 17, comprising:
a waist opening; and
a waist low-stretch region in which a magnitude of force required for stretching the waist low-stretch region by a unit length along a peripheral edge portion of the waist opening is greater than a magnitude of force required for stretching another region by the unit length, wherein
in the up-down direction, the stretch-suppressing means is continuous from the waist low-stretch region to the hook member.

19. The wearable article according to claim 16, wherein
the stretch-suppressing means includes
a central stretch-suppressing portion located in a central portion in the left-right direction, and
a pair of lateral stretch-suppressing portions located respectively on both sides of the central stretch-suppressing portion in the left-right direction.

20. The wearable article according to claim 19, comprising
a pair of leg openings, wherein
each of the pair of leg openings includes an overcast-stitched portion along a peripheral edge thereof, and
there is a portion in which the lateral stretch-suppressing portion and the overcast-stitched portion intersect with one another.

21. The wearable article according to any one of claims 2 to 4, comprising:
a pair of leg openings;
a crotch region located between an upper end of each of the leg openings and a lower end of the wearable article in the up-down direction and between respective inner ends of the pair of leg openings in the left-right direction; and
a waist region located between the upper end of each of the leg openings and the upper end of the wearable article in the up-down direction, wherein
in the natural state, a first imaginary line has no portion overlapping the hook member, the first imaginary line connecting
an end on an upper side of the waist region on one side in the left-right direction and
an end on a lower side of the crotch region on the one side in the left-right direction.

22. The wearable article according to claim 21, comprising
a central stretch-suppressing portion located in a central portion in the left-right direction and having a predetermined length in the up-down direction, wherein
in the natural state, a second imaginary line has no portion overlapping the hook member, the second imaginary line connecting
the end on the upper side of the waist region on the one side in the left-right direction and
a lower end of the central stretch-suppressing portion.

23. The wearable article according to claim 22, comprising
the central stretch-suppressing portion located in the central portion in the left-right direction and having the predetermined length in the up-down direction, wherein
in the natural state, a third imaginary line has no portion overlapping the hook member, the third imaginary line connecting
the end on the upper side of the waist region on the one side in the left-right direction and
an end on the lower side of the crotch region on an other side in the left-right direction.

24. An absorbent pad to be used in combination with the wearable article according to any one of claims 2 to 4,
the absorbent pad having a longitudinal direction, a lateral direction, and a thickness direction intersecting with one another in a stretched state,
the absorbent pad comprising:
a liquid-absorbent absorbent body; and
an other hook member of either one of the male hook member and the female hook member.

25. The absorbent pad according to claim 24, wherein
when viewed in the thickness direction, the hook member has a portion overlapping the absorbent body.

26. The absorbent pad according to claim 25, wherein
the hook member is joined to the absorbent pad by using an adhesive, and
there is no dry edge along a peripheral edge of the hook member.

27. The absorbent pad according to claim 24, wherein
a length in the longitudinal direction is 300 mm or greater, and a length in the lateral direction is 80 mm or greater.
